# EUROPEAN PATENT APPLICATION

(11) **EP 0 860 435 A1**
(43) Date of publication of application: **26.08.1998**
(21) Application number: 98102908.5
(22) Date of filing: 19.02.1998
(51) Int. Cl.: C07D 237/14, C07D 405/12, A01N 43/58

(54) **Pyridazin-3-one derivatives and their use**

(30) Priority: 19.02.1997 JP 35395/97
(71) Applicant: Sumitomo Chemical Company, Limited, Osaka-shi, Osaka-fu (JP)
(72) Inventor: Komori, Takashi, Takarazuka-shi, Hyogo-ken (JP); Hoshi, Hisayuki, Toyonaka-shi, Osaka-fu (JP); Enomoto, Masayuki, Takarazuka-shi, Hyogo-ken (JP)
(74) Representative: VOSSIUS & PARTNER

(57) **Abstract**

The present invention provides novel pyridazin-3-one derivatives of general formula (1): 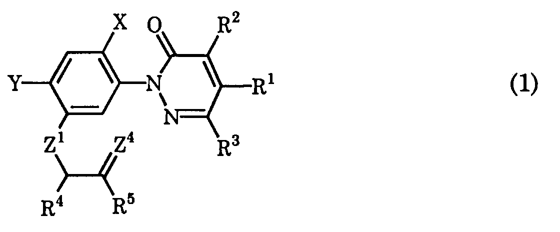 wherein R¹ is haloalkyl; R² and R³ are independently hydrogen, alkyl, haloalkyl, or alkoxyalkyl; X is hydrogen or halogen; Y is halogen, nitro, cyano, or trihalomethyl; Z¹ is oxygen, sulfur, CH₂, or NH; Z⁴ is oxygen or sulfur; R⁴ is hydrogen, alkyl, or cycloalkyl; and R⁵ is alkenyl, haloalkenyl, alkynyl, haloalkynyl, or other substituents as defined in the description, and herbicides containing them as active ingredients.

## Description

The present invention relates to pyridazin-3-one derivatives having excellent herbicidal activity and their use.

The present inventors have extensively studied to seek out some compounds having excellent herbicidal activity. As a result, they have found that pyridazin-3-one derivatives of general formula (1) as depicted below have excellent herbicidal activity, thereby completing the present invention.

Thus, the present invention provides pyridazin-3-one derivatives of general formula (1): 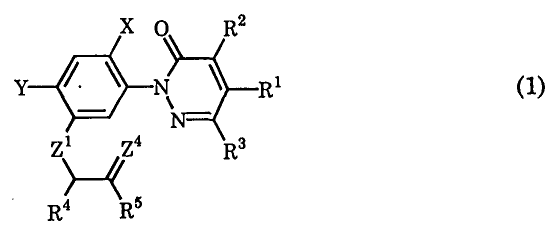 wherein
R¹ is C₁-C₃ haloalkyl;
R² and R³ are the same or different, and are independently hydrogen, C₁-C₃ alkyl, C₁-C₃ haloalkyl, or C₁-C₃ alkoxy C₁-C₃ alkyl;
X is hydrogen or halogen;
Y is halogen, nitro, cyano, or trihalomethyl;
Z¹ is oxygen, sulfur, CH₂, or NH;
Z⁴ is oxygen or sulfur;
R⁴ is hydrogen, C₁-C₆ alkyl, or C₃-C₆ cycloalkyl; and
R⁵ is C₂-C₆ alkenyl, C₂-C₆ haloalkenyl, C₃-C₆ alkynyl, C₃-C₆ haloalkynyl, optionally substituted benzyl, optionally substituted phenyl, C₂-C₆ alkenyloxy, C₂-C₆ haloalkenyloxy, C₃-C₆ alkynyloxy, C₃-C₆ haloalkynyloxy, C₁-C₆ alkylthio, C₁-C₆ haloalkylthio, C₂-C₆ alkenylthio, C₂-C₆ haloalkenylthio, (C₁-C₆ alkyl)thio C₁-C₆ alkyl, C₃-C₆ alkynylthio, C₃-C₆ haloalkynylthio, C₃-C₈ cycloalkylthio, C₃-C₈ cyclohaloalkylthio, (C₃-C₈ cycloalkyl) C₁-C₆ alkylthio, di(C₁-C₆ alkyl)C=NO, optionally substituted benzylthio, optionally substituted phenylthio, hydroxy C₂-C₆ alkoxy, amino C₂-C₆ alkoxy, (C₁-C₆ alkyl)carbonyloxy C₂-C₆ alkoxy, (C₁-C₆ alkyl)carbonylamino C₂-C₆ alkoxy, optionally substituted benzyloxy, optionally substituted phenoxy, optionally substituted furyloxy, optionally substituted furyl C₁-C₆ alkyloxy, optionally substituted thienyloxy, optionally substituted thienyl C₁-C₆ alkyloxy, optionally substituted pyrrolyloxy, optionally substituted pyrrolyloxy C₁-C₆ alkyloxy, optionally substituted imidazolyloxy, optionally substituted imidazolyl C₁-C₆ alkyloxy, optionally substituted pyrazolyloxy, optionally substituted pyrazolyl C₁-C₆ alkyloxy, optionally substituted thiazolyloxy, optionally substituted thiazolyl C₁-C₆ alkyloxy, optionally substituted oxazolyloxy, optionally substituted oxazolyl C₁-C₆ alkyloxy, optionally substituted isothiazolyloxy, optionally substituted isothiazolyl C₁-C₆ alkyloxy, optionally substituted isoxazolyloxy, optionally substituted isoxazolyl C₁-C₆ alkyloxy, optionally substituted pyridyloxy, optionally substituted pyridyl C₁-C₆ alkyloxy, optionally substituted pyrazinyloxy, optionally substituted pyrazinyl C₁-C₆ alkyloxy, optionally substituted pyrimidinyloxy, optionally substituted pyrimidinyl C₁-C₆ alkyloxy, optionally substituted pyridazinyloxy, optionally substituted pyridazinyl C₁-C₆ alkyloxy, optionally substituted indolidinyloxy, optionally substituted indolidinyl C₁-C₆ alkyloxy, optionally substituted indolyloxy, optionally substituted indolyl C₁-C₆ alkyloxy, optionally substituted indazolyloxy, optionally substituted indazolyl C₁-C₆ alkyloxy, optionally substituted quinolyloxy, optionally substituted quinolyl C₁-C₆ alkyloxy, optionally substituted isoquinolyloxy, or optionally substituted isoquinolyl C₁-C₆ alkyloxy, or
a group of -NR⁶R⁷ wherein R⁶ is hydrogen, C₃-C₈ cycloalkyl, (C₁-C₆ alkyl) carbonyloxy C₂-C₆ alkyl, (C₁-C₆ alkyl)carbonylamino C₂-C₆ alkyl, hydroxy C₂-C₆ alkyl, amino C₂-C₆ alkyl, optionally substituted benzyl, or optionally substituted phenyl, and R⁷ is C₃-C₈ cycloalkyl, (C₁-C₆ alkyl)carbonyloxy C₂-C₆ alkyl, (C₁-C₆ alkyl)carbonylamino C₂-C₆ alkyl, hydroxy C₂-C₆ alkyl, amino C₂-C₆ alkyl, optionally substituted benzyl, or optionally substituted phenyl; or R⁶ and R⁷ are taken together to form trimethylene or ethylenethioethylene, or
a group of -NHNHR⁸ wherein R⁸ is hydrogen, C₁-C₆ alkyl, optionally substituted phenyl, or optionally substituted benzyl, or
a group of general formula (2): 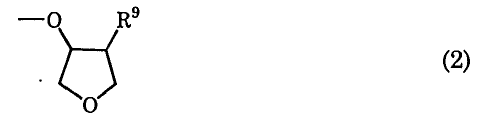 wherein R⁹ is hydrogen, hydroxyl, or a group of -O-COR¹⁰, and R¹⁰ is C₁-C₆ alkyl, C₁-C₆ haloalkyl, C₂-C₆ alkenyl, C₃-C₈ cycloalkyl, optionally substituted phenyl, optionally substituted benzyl, C₁-C₆ alkoxy, or C₁-C₆ alkylamino (hereinafter referred to as the present compound(s)), and herbicides containing them as active ingredients.

Particular examples of the present compounds are as follows:
pyridazin-3-one derivatives wherein Z¹ is oxygen, sulfur, or NH, and R⁵ is C₂-C₆ alkenyl, C₂-C₆ haloalkenyl, C₃-C₆ alkynyl, C₃-C₆ haloalkynyl, optionally substituted benzyl, optionally substituted phenyl, C₁-C₆ alkylthio, C₁-C₆ haloalkylthio, C₂-C₆ alkenylthio, C₂-C₆ haloalkenylthio, (C₁-C₆ alkyl)thio C₁-C₆ alkyl, C₃-C₆ alkynylthio, C₃-C₆ haloalkynylthio, C₃-C₈ cycloalkylthio, C₃-C₈ cyclohaloalkylthio, (C₃-C₈ cycloalkyl) C₁-C₆ alkylthio, di(C₁-C₆ alkyl)C=NO, optionally substituted benzylthio, optionally substituted phenylthio, hydroxy C₂-C₆ alkoxy, amino C₂-C₆ alkoxy, (C₁-C₆ alkyl)carbonyloxy C₂-C₆ alkoxy, (C₁-C₆ alkyl)carbonylamino C₂-C₆ alkoxy, optionally substituted furyloxy, optionally substituted furyl C₁-C₆ alkyloxy, optionally substituted thienyloxy, optionally substituted thienyl C₁-C₆ alkyloxy, optionally substituted pyrrolyloxy, optionally substituted pyrrolyloxy C₁-C₆ alkyloxy, optionally substituted imidazolyloxy, optionally substituted imidazolyl C₁-C₆ alkyloxy, optionally substituted pyrazolyloxy, optionally substituted pyrazolyl C₁-C₆ alkyloxy, optionally substituted thiazolyloxy, optionally substituted thiazolyl C₁-C₆ alkyloxy, optionally substituted oxazolyloxy, optionally substituted oxazolyl C₁-C₆ alkyloxy, optionally substituted isothiazolyloxy, optionally substituted isothiazolyl C₁-C₆ alkyloxy, optionally substituted isoxazolyloxy, optionally substituted isoxazolyl C₁-C₆ alkyloxy, optionally substituted pyridyloxy, optionally substituted pyridyl C₁-C₆ alkyloxy, optionally substituted pyrazinyloxy, optionally substituted pyrazinyl C₁-C₆ alkyloxy, optionally substituted pyrimidinyloxy, optionally substituted pyrimidinyl C₁-C₆ alkyloxy, optionally substituted pyridazinyloxy, optionally substituted pyridazinyl C₁-C₆ alkyloxy, optionally substituted indolidinyloxy, optionally substituted indolidinyl C₁-C₆ alkyloxy, optionally substituted indolyloxy, optionally substituted indolyl C₁-C₆ alkyloxy, optionally substituted indazolyloxy, optionally substituted indazolyl C₁-C₆ alkyloxy, optionally substituted quinolyloxy, optionally substituted quinolyl C₁-C₆ alkyloxy, optionally substituted isoquinolyloxy, optionally substituted isoquinolyl C₁-C₆ alkyloxy, a group of -NR⁶R⁷wherein R⁶ and R⁷ are as defined above, a group of -NHNHR⁸ wherein
R⁸ is as defined above, or a group of general formula 2 as depicted above;
pyridazin-3-one derivatives wherein R¹ is trifluoromethyl, R² is methyl or hydrogen, R³ is hydrogen, X is fluorine, Y is chlorine, R⁴ is hydrogen or methyl, and R⁵ is C₂-C₆ alkenyloxy, C₂-C₆ haloalkenyloxy, C₃-C₆ alkynyloxy, C₃-C₆ haloalkynyloxy, optionally substituted benzyloxy, or optionally substituted phenoxy;
pyridazin-3-one derivatives wherein Z⁴ is oxygen;
pyridazin-3-one derivatives wherein Z¹ and Z⁴ are both oxygen;
pyridazine-3-one derivatives wherein R⁵ is C₂-C₆ alkenyloxy, C₃-C₆ alkynyloxy, benzyloxy, or phenoxy;
pyridazin-3-one derivatives wherein R⁵ is C₂-C₆ alkenyloxy;
pyridazin-3-one derivatives wherein R⁵ is benzyloxy;
pyridazin-3-one derivatives wherein R⁵ is phenoxy;
pyridazin-3-one derivatives wherein R² is methyl;
pyridazin-3-one derivatives wherein Y is halogen, R¹ is trifluoromethyl, R² is hydrogen or methyl, R³ is hydrogen, and R⁴ is hydrogen or methyl;
pyridazin-3-one derivatives wherein X is fluorine, Y is halogen, R¹ is trifluoromethyl, R² is hydrogen or methyl, R³ is hydrogen, and R⁴ is hydrogen or methyl;
pyridazin-3-one derivatives wherein X is fluorine, Y is chlorine, R¹ is trifluoromethyl, R² is hydrogen or methyl, R³ is hydrogen, and R⁴ is hydrogen or methyl;
pyridazin-3-one derivatives wherein X is fluorine, Y is chlorine, R¹ is trifluoromethyl, R² is hydrogen or methyl, R³ is hydrogen, and R⁴ is methyl;
pyridazin-3-one derivatives wherein R⁵ is NHR⁷, and R⁷ is (C₁-C₆ alkyl)carbonyloxy C₂-C₆ alkyl, hydroxy C₂-C₆ alkyl, benzyl, or phenyl;
pyridazin-3-one derivatives wherein R₅ is azetidin-1-yl or thiomorpholin-1-yl; and
pyridazin-3-one derivatives wherein R⁵ is a group of general formula 2 as depicted above, and R⁹ is hydroxyl or (C₁-C₆ alkyl)carbonyloxy.

In the present invention, substituents in the "optionally substituted" groups may include C₁-C₄ alkyl such as methyl, ethyl, propyl, and isopropyl;
C₁-C₄ haloalkyl such as chloromethyl, bromomethyl, and trifluoromethyl;
C₁-C₄ alkoxy such as methoxy, ethoxy, propoxy, and isopropoxy;
(C₁-C₄ alkyl)carbonyloxy such as acetyloxy and ethylcarbonyloxy;
(C₁-C₄ alkoxy)carbonyl such as methoxycarbonyl, ethoxycarbonyl, propoxycarbonyl, and isopropoxycarbonyl;
C₁-C₄ haloalkoxy such as chloromethoxy, bromomethoxy, and trifluoromethoxy;
(C₁-C₄ haloalkyl)carbonyloxy such as chloroacetyloxy and trifluoroacetyloxy;
(C₁-C₄ haloalkoxy)carbonyl such as chloroethoxycarbonyl, bromoethoxycarbonyl, and trifluoroethoxycarbonyl;
halogen such as chlorine, fluorine, and bromine;
nitro, cyano, hydroxyl, amino, and acetyl.

The C₁-C₃ haloalkyl represented by R¹ may include trifluoromethyl, chlorodifluoromethyl, and pentafluoroethyl.

The C₁-C₃ alkyl represented by R² or R³ may include methyl, ethyl, and isopropyl.

The C₁-C₃ haloalkyl represented by R² or R³ may include trichloromethyl, trifluoromethyl, difluoromethyl, chlorodifluoromethyl, and pentafluoroethyl.

The C₁-C₃ alkoxy C₁-C₃ alkyl represented by R² or R³ may include methoxymethyl.

The halogen represented by X or Y may include chlorine, fluorine, and bromine.

The trihalomethyl represented by Y may include trifluoromethyl and trichloromethyl.

The C₁-C₆ alkyl represented by R⁴ may include methyl and ethyl.

The C₃-C₆ cycloalkyl represented by R⁴ may include cyclopropyl.

The C₂-C₆ alkenyl represented by R⁵ may include vinyl, allyl, 1-propenyl, 1-methyl-2-propenyl, 2-methyl-2-propenyl, 2-butenyl, and 3-butenyl.

The C₂-C₆ haloalkenyl represented by R⁵ may include 2-chloro-2-propenyl, 3-chloro-2-propenyl, and 3,3-dichloro-2-propenyl.

The C₃-C₆ alkynyl represented by R⁵ may include propargyl, 1-methyl-2-propynyl, 1,1-dimethyl-2-propynyl, 2-butynyl, 3-butynyl, and 1,2-dimethyl-3-butynyl.

The C₃-C₆ haloalkynyl represented by R⁵ may include 3-bromo-2-propynyl.

The optionally substituted benzyl represented by R⁵ may include benzyl, α-methylbenzyl, α,α-dimethylbenzyl, and 4-methoxybenzyl.

The optionally substituted phenyl represented by R⁵ may include phenyl, 4-methylphenyl, 4-methoxyphenyl, 4-trifluoromethylphenyl, and 3-methylphenyl.

The C₂-C₆ alkenyloxy represented by R⁵ may include vinyloxy, allyloxy, 1-propenyloxy, 1-methyl-2-propenyloxy, 2-methyl-2-propenyloxy, 3-methyl-2-butenyloxy, and 3-butenyloxy.

The C₂-C₆ haloalkenyloxy represented by R⁵ may include 2-chloro-2-propenyloxy, 3-chloro-2-propenyloxy, and 3,3-dichloro-2-propenyloxy.

The C₃-C₆ alkynyloxy represented by R⁵ may include propargyloxy, 1-methyl-2-propynyloxy, 1,1-dimethyl-2-propynyloxy, 2-butynyloxy, 3-butynyloxy, and 1,2-dimethyl-3-butynyloxy.

The C₃-C₆ haloalkynyloxy represented by R⁵ may include 2-bromo-2-propynyloxy.

The C₁-C₆ alkylthio represented by R⁵ may include methylthio, ethylthio, n-propylthio, isopropylthio, n-butylthio, isobutylthio, sec-butylthio, and t-butylthio.

The C₁-C₆ haloalkylthio represented by R⁵ may include chloromethylthio, trifluoromethylthio, 2,2-dichloroethylthio, 2,2,2-trifluoroethylthio, and 3-chloropropylthio.

The C₂-C₆ alkenylthio represented by R⁵ may include vinylthio, allylthio, 1-propenylthio, 1-methyl-2-propenylthio, 2-methyl-2-propenylthio, 2-butenylthio, and 3-butenylthio.

The C₂-C₆ haloalkenylthio represented by R⁵ may include 2-chloro-2-propenylthio, 3-chloro-2-propenylthio, and 3,3-dichloro-2-propenylthio.

The (C₁-C₆ alkyl)thio C₁-C₆ alkyl represented by R⁵ may include methylthiomethyl, ethylthiomethyl, isopropylthiomethyl, and methylthioethyl.

The C₃-C₆ alkynylthio represented by R⁵ may include propargylthio, 1-methyl-2-propynylthio, 1,1-dimethyl-2-propynylthio, 2-butynylthio, 3-butynylthio, and 1,2-dimethyl-3-butynylthio.

The C₃-C₆ haloalkynylthio represented by R⁵ may include 2-bromo-2-propynylthio.

The C₃-C₈ cycloalkylthio represented by R⁵ may include cyclopropylthio, cyclobutylthio, cyclopentylthio, cyclohexylthio, and cycloheptylthio.

The optionally substituted benzylthio represented by R⁵ may include benzylthio and 4-methylbenzylthio.

The optionally substituted phenylthio represented by R⁵ may include phenylthio and 4-methylphenylthio.

The hydroxy C₂-C₆ alkoxy represented by R⁵ may include 2-hydroxyethoxy, 3-hydroxypropoxy, 1-methyl-2-hydroxyethoxy, 2-hydroxypropoxy, and 1-methyl-2-hydroxypropoxy.

The amino C₂-C₆ alkoxy represented by R⁵ may include 2-aminoethoxy, 3-aminopropoxy, 1-methyl-2-aminoethoxy, 2-aminopropoxy, and 1-methyl-2-aminopropoxy.

The (C₁-C₆ alkyl)carbonyloxy C₂-C₆ alkoxy represented by R⁵ may include 2-acetoxyethoxy, 2-ethylcarbonyloxyethoxy, 2-isopropylcarbonyloxyethoxy, 2-(t-butylcarbonyloxy)ethoxy, and 3-acetoxypropoxy.

The (C₁-C₆ alkyl)carbonylamino C₂-C₆ alkoxy represented by R⁵ may include 2-methylcarbonylaminoethoxy, 2-ethylcarbonylaminoethoxy, 2-isopropylcarbonylaminoethoxy, 2-(t-butylcarbonylamino)ethoxy, and 3-methylcarbonylaminopropoxy.

The optionally substituted benzyloxy represented by R⁵ may include benzyloxy, α-methylbenzyloxy, 4-trifluoromethylbenzyloxy, 4-methoxybenzyloxy, 2-chlorobenzyloxy, 4-chlorobenzyloxy, 2,6-dichlorobenzyloxy, and 4-nitrobenzyloxy.

The optionally substituted phenoxy represented by R⁵ may include phenoxy, 4-methylphenoxy, and 4-nitrophenoxy.

The optionally substituted furyl C₁-C₆ alkyloxy represented by R⁵ may include 2-furylmethoxy, and 3-furylmethoxy.

The C₃-C₈ cycloalkyl represented by R⁶ or R⁷ may include cyclopropyl, cyclobutyl, cyclopentyl, cyclohexyl and cycloheptyl.

The (C₁-C₆ alkyl)carbonyloxy C₂-C₆ alkyl represented by R⁶ or R⁷ may include 2-acetoxyethyl, 2-ethylcarbonyloxyethyl, 2-isopropylcarbonyloxyethyl, 2-(t-butylcarbonyloxy)ethyl, and 3-acetoxypropyl.

The (C₁-C₆ alkyl)carbonylamino C₂-C₆ alkyl represented by R⁶ or R⁷ may include 2-methylcarbonylaminoethyl, 2-ethylcarbonylaminoethyl, 2-isopropylcarbonylaminoethyl, 2-(t-butylcarbonylamino)ethyl, and 3-methylcarbonylaminopropyl.

The hydroxy C₂-C₆ alkyl represented by R⁶ or R⁷ may include 2-hydroxyethyl, 3-hydroxypropyl, 1-methyl-2-hydroxyethyl, and 2-hydroxypropyl.

The amino C₂-C₆ alkyl represented by R⁶ or R⁷ may include 2-aminoethyl, 3-aminopropyl, 1-methyl-2-aminoethyl, and 2-aminopropyl.

The optionally substituted benzyl represented by R⁶ or R⁷ may include benzyl, α-methylbenzyl, α,α-dimethylbenzyl, and 4-methylbenzyl.

The optionally substituted phenyl represented by R⁶ or R⁷ may include phenyl, 4-methylphenyl, 4-methoxyphenyl, 4-trifluromethylphenyl, and 3-methylphenyl.

The C₁-C₆ alkyl represented by R⁸ may include methyl, ethyl, n-propyl, isopropyl, n-butyl, isobutyl, sec-butyl, and t-butyl.

The optionally substituted phenyl represented by R⁸ may include phenyl, 4-methylphenyl, 4-methoxyphenyl, 4-trifluoromethylphenyl, and 3-methylphenyl.

The optionally substituted benzyl represented by R⁸ may include benzyl, α-methylbenzyl, α,α-dimethylbenzyl, and 4-methylbenzyl.

The C₁-C₆ alkyl represented by R¹⁰ may include methyl, ethyl, n-propyl, isopropyl, n-butyl, isobutyl, sec-butyl, and t-butyl.

The C₁-C₆ haloalkyl represented by R¹⁰ may include chloromethyl, trifluoromethyl, 2,2-dichloroethyl, 2,2,2-trifluoloethyl, and 3-chloropropyl.

The C₂-C₆ alkenyl represented by R¹⁰ may include vinyl, allyl, 1-propenyl, 1-methyl-2-propenyl, 2-methyl-2-propenyl, 2-butenyl, and 3-butenyl.

The C₃-C₈ cycloalkyl represented by R¹⁰ may include cyclopropyl, cyclobutyl, cyclopentyl, cyclohexyl, and cycloheptyl.

The optionally substituted phenyl represented by R¹⁰ may include phenyl, 4-methylphenyl, 4-methoxyphenyl, 4-trifluoromethylphenyl, and 3-methylphenyl.

The optionally substituted benzyl represented by R¹⁰ may include benzyl, α-methylbenzyl, α,α-dimethylbenzyl, and 4-methylbenzyl.

The C₁-C₆ alkoxy represented by R¹⁰ may include methoxy, ethoxy, propoxy, isopropoxy, n-butoxy, sec-butoxy, isobutoxy, and t-butoxy.

The C₁-C₆ alkylamino represented by R¹⁰ may include methylamino, ethylamino, isopropylamino, and n-propylamino.

In the present compounds, preferred examples of the substituents in view of herbicidal activity are as follows:
fluorine and chlorine for X;
chlorine for Y;
oxygen for Z⁴;
a group of CF₂Z² wherein Z² is hydrogen, fluorine, or trifluoromethyl, for R¹, more preferably trifluoromethyl;
methyl and hydrogen for R²;
methyl and hydrogen for R³; and
methyl and hydrogen for R⁴.

Preferred examples of the present compounds in view of herbicidal activity are those having the above preferred substituents in combination. Particularly preferred examples are those wherein R¹ is trifluoromethyl, R² is methyl or hydrogen, R³ is hydrogen, X is fluorine, Y is chlorine, Z¹ is oxygen, Z⁴ is oxygen, R⁴ is methyl or hydrogen, and R⁵ is vinyloxy, phenoxy, or benzyloxy.

For the present compounds, there may exist optical isomers based on the presence of at least one asymmetric carbon atom, and all of these optical isomers are, of course, included within the scope of the present invention.

The present compounds can be produced, for example, by Production Processes 1 to 3 as described below.

### (Production Process 1)

This is the production process according to the following scheme 1: 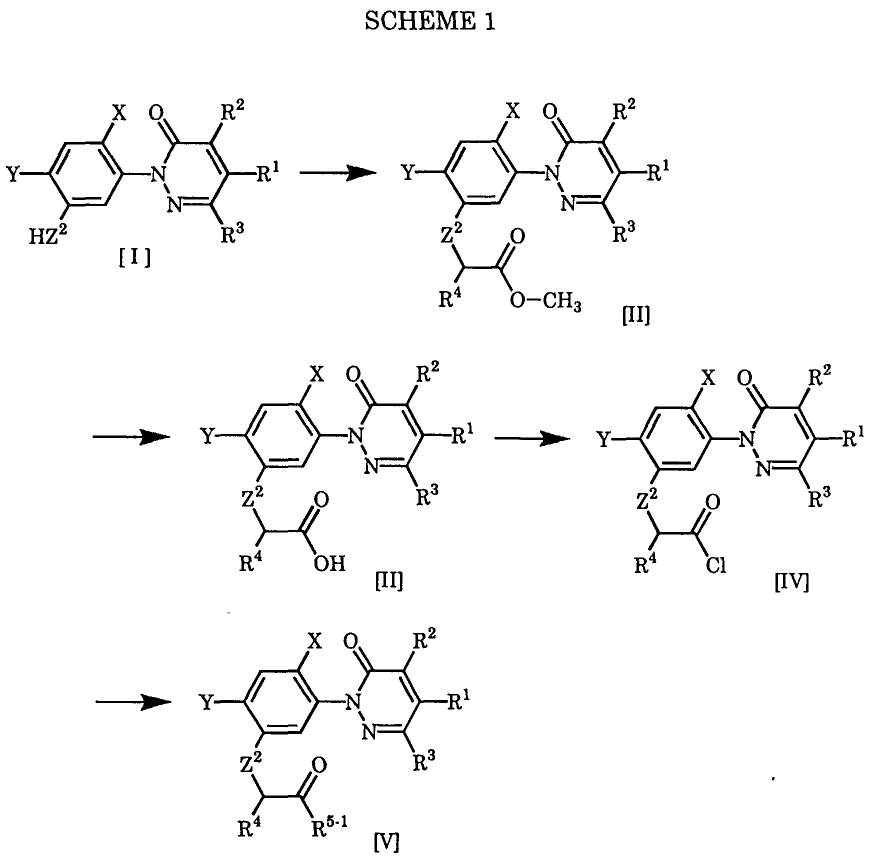 wherein X, Y R¹, R², R³, and R⁴ are as defined above, Z² is oxygen, sulfur, or NH, and R⁵⁻¹ is C₃-C₆ alkenyloxy, C₃-C₆ haloalkenyloxy, C₃-C₆ alkynyloxy, C₃-C₆ haloalkynyloxy, C₁-C₆ alkylthio, C₁-C₆ haloalkylthio, C₃-C₆ alkenylthio, C₃-C₆ haloalkenylthio, C₃-C₆ alkynylthio, C₃-C₆ haloalkynylthio, C₃-C₈ cycloalkylthio, C₃-C₈ cyclohaloalkylthio, (C₃-C₈ cycloalkyl) C₁-C₆ alkylthio, di(C₁-C₆ alkyl)C=NO, optionally substituted benzylthio, optionally substituted phenylthio, hydroxy C₂-C₆ alkoxy, amino C₂-C₆ alkoxy, (C₁-C₆ alkyl)carbonyloxy C₂-C₆ alkoxy, (C₁-C₆ alkyl)carbonylamino C₂-C₆ alkoxy, optionally substituted benzyloxy, optionally substituted phenoxy, optionally substituted furyloxy, optionally substituted furyl C₁-C₆ alkyloxy, optionally substituted thienyloxy, optionally substituted thienyl C₁-C₆ alkylthio, optionally substituted pyrrolyloxy, optionally substituted pyrrolyloxy C₁-C₆ alkyloxy, optionally substituted imidazolyloxy, optionally substituted imidazolyl C₁-C₆ alkyloxy, optionally substituted pyrazolyloxy, optionally substituted pyrazolyl C₁-C₆ alkyloxy, optionally substituted thiazolyloxy, optionally substituted thiazolyl C₁-C₆ alkyloxy, optionally substituted oxazolyloxy, optionally substituted oxazolyl C₁-C₆ alkyloxy, optionally substituted isothiazolyloxy, optionally substituted isothiazolyl C₁-C₆ alkyloxy, optionally substituted isoxazolyloxy, optionally substituted isoxazolyl C₁-C₆ alkyloxy, optionally substituted pyridyloxy, optionally substituted pyridyl C₁-C₆ alkyloxy, optionally substituted pyrazinyloxy, optionally substituted pyrazinyl C₁-C₆ alkyloxy, optionally substituted pyrimidinyloxy, optionally substituted pyrimidinyl C₁-C₆ alkyloxy, optionally substituted pyridazinyloxy, optionally substituted pyridazinyl C₁-C₆ alkyloxy, optionally substituted indolidinyloxy, optionally substituted indolidinyl C₁-C₆ alkyloxy, optionally substituted indolyloxy, optionally substituted indolyl C₁-C₆ alkyloxy, optionally substituted indazolyloxy, optionally substituted indazolyl C₁-C₆ alkyloxy, optionally substituted quinolyloxy, optionally substituted quinolyl C₁-C₆ alkyloxy, optionally substituted isoquinolyloxy, optionally substituted isoquinolyl C₁-C₆ alkyloxy, a group of -NR⁶R⁷ wherein R⁶ and R⁷ are as defined above, a group of -NHNHR⁸ wherein R⁸ is as defined above, or a group of general formula 2 as depicted above.

The reaction conditions in each step are, for example, as follows:

### Step of producing compound [II] from compound [I]

Compound [II] can be produced by reacting compound [ I ] with a compound of the general formula:

CHZ(R⁴)COOCH₃

wherein Z is bromine or chlorine, and R⁴ is as defined above, in the presence of a base, if necessary.

The reaction is effected without solvent or in a solvent. The reaction temperature is usually in the range of -20° to 150°C. The reaction time is usually in the range of a moment to 72 hours. The amounts of reagents to be used in the reaction are 1 mole of compound CHZ(R⁴)COOCH₃ and 1 mole of the base used, if necessary, relative to 1 mole of compound [ I ], which is the stoichiometric ratio but can be freely changed depending upon the reaction conditions.

The solvent which can be used may include aliphatic hydrocarbons such as hexane, cyclohexane, and petroleum ether; aromatic hydrocarbons such as benzene, toluene, and xylene; ethers such as diethyl ether, 1,4-dioxane, and tetrahydrofuran (hereinafter referred to as THF); acid amides such as formamide and N,N-dimethylformamide; tertiary amines such as pyridine, triethylamine, and N,N-dimethylaniline; sulfur compounds such as dimethylsulfoxide and sulforane; and mixtures thereof.

The base which can be used, if necessary, may include inorganic bases such as sodium hydrogencarbonate, potassium hydrogencarbonate, sodium carbonate, potassium carbonate, sodium hydroxide, potassium hydroxide, and sodium hydride; organic bases such as triethylamine, diisopropylethylamine, pyridine, 4-dimethylaminopyridine, picoline, and N,N-dimethylaniline; and mixtures thereof.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

### Step of producing compound [III] from compound [II]

Compound [III] can be produced by hydrolyzing compound [II] in the presence of one or more acids. The reaction is usually effected in a solvent. The reaction temperature is usually in the range of 0° to 100°C. The reaction time is usually in the range of a moment to 72 hours.

The solvent which can be used may include aliphatic hydrocarbons such as hexane, cyclohexane, and petroleum ether; aromatic hydrocarbons such as benzene, toluene, and xylene; ethers such as diethyl ether, 1,4-dioxane, and THF; nitro compounds such as nitromethane and nitrobenzene; sulfur compounds such as dimethylsulfoxide and sulforane; alcohols such as methanol, ethanol, and ethylene glycol; halogenated hydrocarbons such as chloroform, dichloromethane, and chlorobenzene; nitriles such as acetonitrile and benzonitrile; fatty acids such as formic acid, acetic acid, and propionic acid; water; and mixtures thereof.

The acid which can be used may include mineral acids such as hydrochloric acid, sulfuric acid, and nitric acid; organic acids such as acetic acid, propionic acid, and p-toluenesulfonic acid; and mixtures thereof. The acid is used at an amount ranging from a catalytic amount to a large excess relative to compound [II].

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

### Step of producing compound [IV] from compound [III]

Compound [IV] can be produced by reacting compound [III] with a chlorinating agent. The reaction is effected without solvent or in a solvent. The reaction temperature is usually in the range of room temperature to a heating temperature under reflux. The reaction time is usually in the range of a moment to 72 hours.

The chlorinating agent which can be used may include thionyl chloride, phosgene, oxalyl chloride, phosphorus trichloride, phosphorus pentachloride, and phosphorus oxychloride. The amounts of reagents to be used in the reaction are 1 mole of the chlorinating agent relative to 1 mole of compound [III], which is the stoichiometric ratio but can be freely changed depending upon the reaction conditions.

The solvent which can be used in the reaction may include aliphatic hydrocarbons such as hexane, cyclohexane, and petroleum ether; aromatic hydrocarbons such as benzene, toluene, and xylene; ethers such as diethyl ether, 1,4-dioxane, and THF; nitro compounds such as nitromethane and nitrobenzene; halogenated hydrocarbons such as chloroform, dichloromethane, and chlorobenzene; ketones such as acetone, methyl ethyl ketone, and cyclohexanone; esters such as ethyl formate, ethyl acetate, and diethyl carbonate; nitriles such as acetonitrile and benzonitrile; and mixtures thereof.

After completion of the reaction, the solvent is evaporated under reduced pressure. Thus, the desired compound can be isolated.

### Step of producing compound [V] from compound [IV]

Compound [V] can be produced by reacting compound [IV] with a compound of the general formula:

R⁵⁻¹-H

wherein R⁵⁻¹ is as defined above, in the presence of one or more bases without solvent or in a solvent. The reaction temperature is usually in the range of room temperature to a heating temperature under reflux. The reaction time is usually in the range of a moment to 72 hours. The amounts of reagents to be used in the reaction are 1 mole of compound R⁵⁻¹-H and 1 mole of the base relative to 1 mole of compound [IV], which is the stoichiometric ratio but can be freely changed depending upon the reaction conditions.

The base which can be used may include inorganic bases such as sodium hydrogencarbonate, potassium hydrogencarbonate, sodium carbonate, potassium carbonate, sodium hydroxide, potassium hydroxide, and sodium hydride; organic bases such as triethylamine, diisopropylethylamine, pyridine, 4-dimethylaminopyridine, picoline, and N,N-dimethylaniline; and mixtures thereof.

The solvent which can be used may include aliphatic hydrocarbons such as hexane, cyclohexane, and petroleum ether; aromatic hydrocarbons such as benzene, toluene, and xylene; ethers such as diethyl ether, 1,4-dioxane, and THF; acid amides such as formamide and N,N-dimethylformamide; tertiary amines such as pyridine, triethylamine, and N,N-dimethylaniline; sulfur compounds such as dimethylsulfoxide and sulforane; ketones such as acetone, methyl ethyl ketone, and cyclohexanone; esters such as ethyl formate, ethyl acetate, and diethyl carbonate; nitriles such as acetonitrile and benzonitrile; and mixtures thereof.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

### (Production Process 2)

This is the production process according to the following scheme 2: 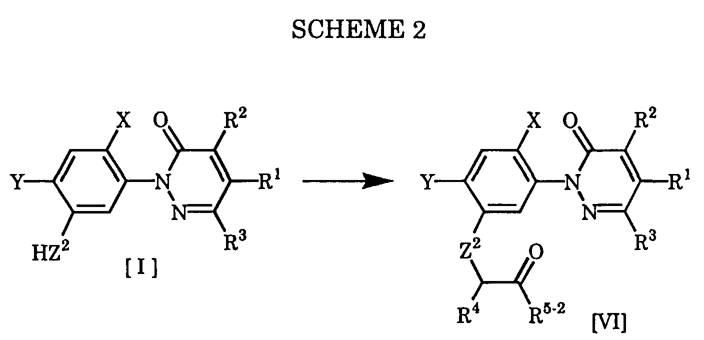 wherein X, Y, R¹, R², R³, R⁴, and Z² are as defined above, and R⁵⁻² is C₂-C₆ alkenyl, C₂-C₆ haloalkenyl, C₃-C₆ alkynyl, C₃-C₆ haloalkynyl, optionally substituted benzyl, optionally substituted phenyl, C₂-C₆ alkenyloxy, C₂-C₆ haloalkenyloxy, C₂-C₆ alkenylthio, C₂-C₆ haloalkenylthio, or (C₁-C₆ alkyl)thio C₁-C₆ alkyl.

The reaction conditions in the above step are, for example, as follows:

Compound [VI] can be produced by reacting compound [ I ] with a compound of the general formula:

CHZ(R⁴)COR⁵⁻²

wherein Z is bromine or chlorine, and R⁴ and R⁵⁻² are as defined above, in the presence of one or more bases, if necessary.

The reaction is effected without solvent or in a solvent. The reaction temperature is usually in the range of -20° to 150°C. The reaction time is usually in the range of a moment to 72 hours.

The solvent which can be used may include aliphatic hydrocarbons such as hexane, cyclohexane, and petroleum ether; aromatic hydrocarbons such as benzene, toluene, and xylene; ethers such as diethyl ether, 1,4-dioxane, and THF; acid amides such as formamide and N,N-dimethylformamide; tertiary amines such as pyridine, triethylamine, and N,N-dimethylaniline; sulfur compounds such as dimethylsulfoxide and sulforane; and mixtures thereof.

The amounts of reagents to be used in the reaction are 1 mole of compound CHZ(R⁴)COR⁵⁻² and 1 mole of the base relative to 1 mole of compound [ I ], which is the stoichiometric ratio but can be freely changed depending upon the reaction conditions.

The base which can be used, if necessary, may include inorganic bases such as sodium hydrogencarbonate, potassium hydrogencarbonate, sodium carbonate, potassium carbonate, sodium hydroxide, potassium hydroxide, and sodium hydride; organic bases such as triethylamine, diisopropylethylamine, pyridine, 4-dimethylaminopyridine, picoline, and N,N-dimethylaniline; and mixtures thereof.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

### (Production Process 3)

This is the production process according to the following scheme 3: 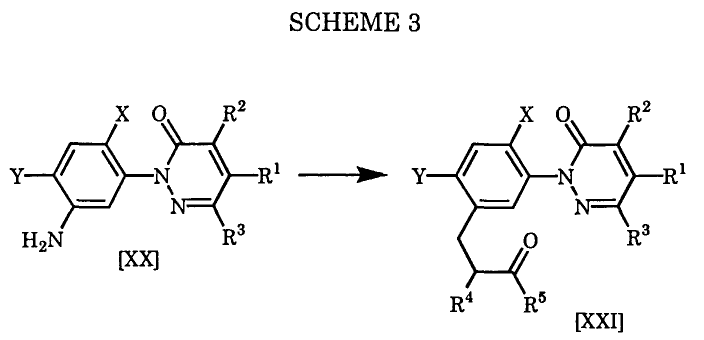 wherein X, Y, R¹, R², R³, R⁴, and R⁵ are as defined above.

The reaction conditions in the above step are, for example, as follows:

Compound [XXI] can be produced by converting compound [XX] to a diazonium salt in a solvent such as hydrochloric acid or bromic acid, and then reacting the diazonium salt with a compound of the general formula:

CH₂=CR⁴COR⁵

wherein R⁴ and R⁵ are as defined above, in a solvent such as acetonitrile.

The reaction temperature is usually in the range of -20° to 150°C, preferably 0° to 60°C. The reaction time is usually in the range of a moment to 72 hours.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

The following will describe the production processes for the starting material compounds used in the production of the present compounds.

Compound [ I ] wherein Z² is oxygen or sulfur can be produced according to the following scheme 4. 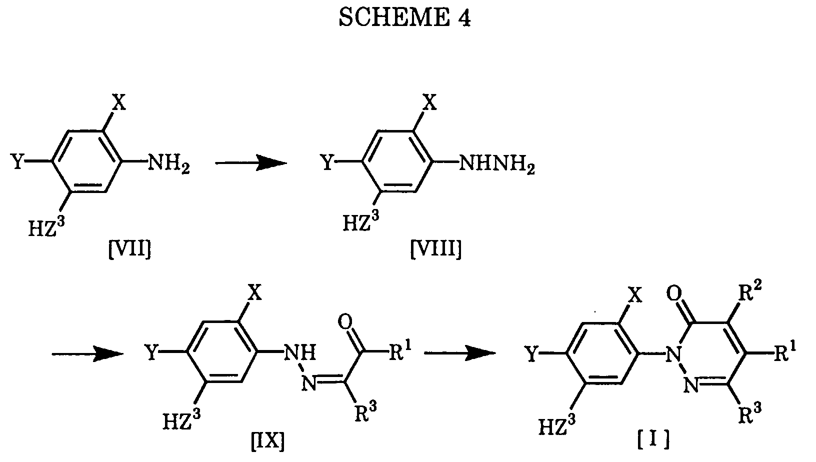 wherein X, Y R¹, R², and R³ are as defined above, and Z³ is oxygen or sulfur.

The aniline derivative designated as compound [VII] in the above scheme is well known in the art, for example, in the published specification of European Patent Application, EP-A-61741, or can be produced according to the process disclosed therein.

### Step of producing compound [VIII] from compound [VII]

Compound [VIII] can be produced from compound [VII], for example, according to the following scheme 5 (see Organic Synthesis Collective, volume 1., p. 442). 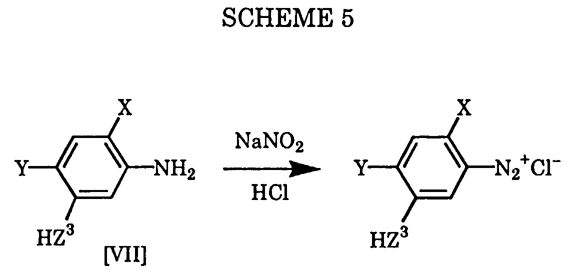 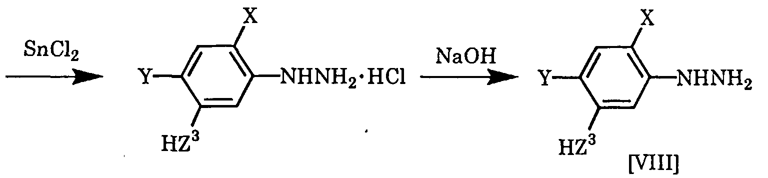 wherein X, Y, and Z³ are as defined above.

### Step of producing compound [IX] from compound [VIII]

Compound [IX] can be produced by reacting an α-dihalo compound of the general formula:

R¹C(=O)CV₂R³

wherein R¹ and R³ are as defined above, and V is iodine, bromine, or chlorine, with water in the presence of a base to give a carbonyl derivative of the general formula:

R¹C(=O)C(=O)R³

wherein R¹ and R³ are as defined above (hereinafter referred to as reaction 1), and then reacting the carbonyl derivative with hydrazine derivative [VIII] (hereinafter referred to as reaction 2). The carbonyl derivative R¹C(=O)C(=O)R³ can also be reacted as a hydrate in water or as an acetal derivative in an alcohol.

The reaction 1 is usually effected in a solvent (e.g., water). The reaction temperature is usually in the range of 0° to 100°C. The reaction time is usually in the range of a moment to 72 hours. The amounts of reagents to be used in the reaction are 2 moles of water and 2 moles of the base (e.g., sodium acetate) relative to 1 mole of α-dihalo compound R¹C(=O)CV₂R³, which is the stoichiometric ratio but can be freely changed depending upon the reaction conditions.

The reaction 2 is usually effected in a solvent (e.g., THF). The reaction temperature is usually in the range of 0° to 100°C. The reaction time is usually in the range of a moment to 72 hours. The amounts of reagents to be used in the reaction are 1 mole of hydrazine derivative [VIII] relative to 1 mole of the α-dihalo compound used in the reaction 1, which is the stoichiometric ratio but can be freely changed depending upon the reaction conditions. The hydrazine derivative [VIII] may also be used in the form of a salt such as hydrochloride or sulfate.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

### Step of producing compound [ I ] from compound [IX]

Compound [ I ] can be produced by reacting compound [IX] with a compound of the general formula:

Ph₃P=C(R²)COOC₂H₅

wherein R² is as defined above and Ph is phenyl.

The above compound Ph₃P=C(R²)COOC₂H₅ is commercially available or can be produced according to the process described, for example, in the "Jikken Kagaku Koza" (published by Maruzen Kabushiki Kaisha), 4th edition, volume 24, pp. 259-260.

The reaction is usually effected in a solvent (e.g., THF). The reaction temperature is usually in the range of -20° to 150°C. The reaction time is usually in the range of a moment to 72 hours. The amounts of reagents to be used in the reactions are 1 mole of compound Ph₃P=C(R²)COOC₂H₅ relative to 1 mole of compound [IX], which is the stoichiometric ratio but can be freely changed depending upon the reaction conditions.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

Compound [ I ] wherein Z² is NH (i.e., compound [XX]) can be produced according to the following scheme. 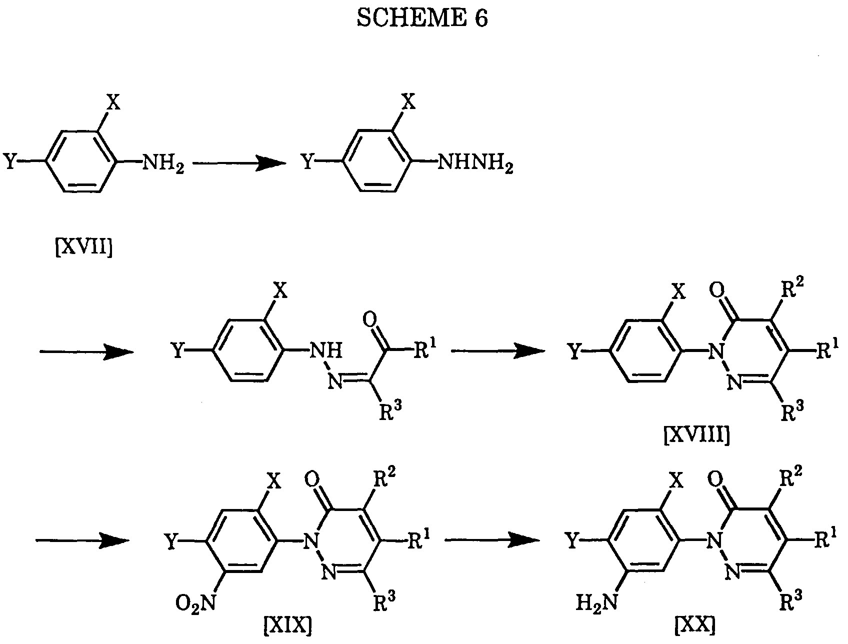 wherein X, Y, R¹, R², and R³ are as defined above.

### Step of producing compound [XVIII] from compound [XVII]

Compound [XVIII] can be produced in the same manner as the process for producing compound [ I ] from compound [VII] (scheme 4).

### Step of producing compound [XIX] from compound [XVIII]

Compound [XIX] can be produced by nitrating compound [XVIII]. The reaction is usually effected in a solvent. The reaction temperature is usually in the range of -10°C to room temperature. The reaction time is usually in the range of a moment to 72 hours.

The nitrating agent may include nitric acid and fuming nitric acid. The amounts of reagents to be used in the reaction are 1 mole of the nitrating agent relative to 1 mole of compound [XVIII], which is the stoichiometric ratio but can be freely changed depending upon the reaction conditions.

The solvent which can be used in the reaction may include aliphatic hydrocarbons such as hexane, cyclohexane, and petroleum ether; acids such as sulfuric acid and acetic acid; and mixtures thereof.

After completion of the reaction, the reaction mixture is poured into ice water and the resulting crystals are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

### Step of producing compound [XX] from compound [XIX]

Compound [XX] can be produced by reducing compound [XIX] with a metal such as iron.

The reaction is usually effected in the presence of an acid in a solvent. The reaction temperature is usually in the range of room temperature to a heating temperature under reflux. The reaction time is usually in the range of a moment to 72 hours.

The reducing agent may include iron, zinc, and tin. The amounts of reagents to be used in the reaction are usually 3 moles to a large excess of the reducing agent relative to 1 mole of compound [XIX].

The solvent which can be used in the reaction may include aliphatic hydrocarbons such as hexane, cyclohexane, and petroleum ether; aromatic hydrocarbons such as benzene, toluene, and xylene; ethers such as diethyl ether, 1,4-dioxane, and THF; esters such as ethyl formate, ethyl acetate, and diethyl carbonate; nitriles such as acetonitrile and benzonitrile; alcohols such as methanol, ethanol, and propanol; water; and mixtures thereof.

The acid which can be used may include acetic acid, hydrochloric acid, and sulfuric acid.

After completion of the reaction, the resulting crystals (deposited by the addition of water, if necessary) are collected by filtration, or the reaction mixture is subjected to ordinary post-treatments such as extraction with an organic solvent and concentration. If necessary, further purification may be carried out by a technique such as chromatography or recrystallization. Thus, the desired compound can be isolated.

The present compounds have excellent herbicidal activity and can exhibit excellent selectivity between crops and weeds.

For example, the present compounds have herbicidal activity against various weeds which may cause some trouble in the foliar treatment and soil treatment on upland fields, such as listed below.

### Polygonaceous weeds:

wild buckwheat (*Polygonum convolvulus*), pale smartweed (*Polygonum lapathiolium*), pennsylvania smartweed (*Polygonum pensylvanicum*), ladysthumb (*Polygonum persicaria*), curly dock (*Rumex crispus*), broadleaf dock (*Rumex obtusifolius*), Japanese knotweed (*Polygonum cuspidatum*)

### Portulacaceous weeds:

common purslane (*Portulaca oleracea*)

### Caryophyllaceous weeds:

common chickweed (*Stellaria media*)

### Chenopodiaceous weeds:

common lambsquarters (*Chenopodium album*), kochia (*Kochia scoparia*)

### Amaranthaceous weeds:

redroot pigweed (*Amaranthus retroflexus*), smooth pigweed (*Amaranthus hybridus*)

### Cruciferous (brassicaceous) weeds:

wild radish (*Raphanus raphanistrum*), wild mustard (*Sinapis arvensis*), shepherdpurse (*Capsella bursa-pastoris*)

### Leguminous (fabaceous) weeds:

hemp sesbania (*Sesbania exaltata*), sicklepod (*Cassia obtusifolia*), Florida beggarweed (*Desmodium tortuosum*), white clover (*Trifolium repen*s)

### Malvaceous weeds:

velvetleaf (*Abutilon theophrasti*), prickly sida (*Sida spinosa*)

### Violaceous weeds:

field pansy (*Viola arvensis*), wild pansy (*Viola tricolor*)

### Rubiaceous weeds:

catchweed bedstraw (cleavers) (*Galium aparine*)

### Convolvulaceous weeds:

ivyleaf morningglory (*Ipomoea hederacea*), tall morningglory (*Ipomoea purpurea*), entireleaf morningglory (*Ipomoea hederacea* var. *integriuscula*), pitted morningglory (*Ipomoea lacunosa*), field bindweed (*Convolvulus arvensis*)

### Labiate weeds:

red deadnettle (*Lamium purpureum*), henbit (*Lamium amplexicaule*)

### Solanaceous weeds:

jimsonweed (*Datura stramonium*), black nightshade (*Solanum nigrum*)

### Scrophulariaceous weeds:

birdseye speedwell (*Veronica persica*), ivyleaf speedwell (*Veronica hederaefolia*)

### Composite weeds:

common cocklebur (*Xanthium pensylvanicum)*, common sunflower (*Helianthus annuus*), scentless chamomile (*Matricaria perforata* or *inodora*), corn marigold (*Chrysanthemum segetum*), pineappleweed (*Matricaria matricarioides*), common ragweed (*Ambrosia artemisiifolia*), giant ragweed (*Ambrosia trifida*), horseweed (*Erigeron canadensis*), Japanese mugwort (*Artemisia princeps*), tall goldenrod (*Solidago altissima*)

### Boraginaceous weeds:

forget-me-not (*Myosotis arvensis*)

### Asclepiadaceous weeds:

common milkweed (*Asclepias syriaca*)

### Euphorbiaceous weeds:

sun spurge (*Euphorbia helioscopia*), spotted spurge (*Euphorbia maculata*)

### Graminaceous weeds:

barnyardgrass (*Echinochloa crus-galli*), green foxtail (*Setaria viridis*), giant foxtail (*Setaria fabert),* large crabgrass (*Digitaria sanguinalis*), goosegrass (*Eleusine indica),* annual bluegrass (*Poa annua*), blackgrass (*Alopecurus myosuroides*), wild oats (*Avena fatua)*, johnsongrass (*Sorghum halepense)*, quackgrass (*Agropyron repens*), downy brome (*Bromus tectorum*), bermudagrass (*Cynodon dactylon*), fall panicum (*Panicum dichotomiflorum)*, Texas panicum (*Panicum texanum*), shattercane (*Sorghum* *vulgare*)

### Commelinaceous weeds:

common dayflower (*Commelina communis*)

### Equisetaceous weeds:

field horsetail (*Equisetum arvense*)

### Cyperaceous weeds:

rice flatsedge (*Cyperus iria*), purple nutsedge (*Cyperus rotundu*s), yellow nutsedge (*Cyperus esculentus*)

Furthermore, some of the present compounds exhibit no significant phytotoxicity on the main crops such as corn (*Zea mays*), wheat (*Titicum aestivum*), barley (*Hordeum vulgare*), rice (*Oryza sativa*), sorghum (*Sorghum bicolor*), soybean (*Glycine max*), cotton (*Gossypium* spp.), sugar beet (*Beta vulgaris)*, peanut (*Arachis hypogaea*), sunflower (*Helianthus annuus*), and canola (*Brassica napus*); horticultural crops such as flowers and ornamental plants; and vegetable crops.

The present compounds can also attain the effective control of various weeds which may cause some trouble in the no-tillage cultivation of soybean (*Glycine max*), corn (*Zea mays*), wheat (*Triticum aestivum*), and other crops. Furthermore, some of the present compounds exhibit no significant phytotoxicity on the crops.

The present compounds also have herbicidal activity against various weeds which may cause some trouble in the flooding treatment on paddy fields, such as listed below.

### Graminaceous weeds:

barnyardgrass (*Echinochloa oryzicola*)

### Scrophulariaceous weeds:

common falsepimpernel (*Lindernia procumbens*)

### Lythraceous weeds:

Indian toothcup (*Rotala indica*), red stem (*Ammannia multiflora*)

### Elatinaceous weeds:

waterwort (*Elatine triandra*)

### Cyperaceous weeds:

smallflower umbrella sedge (*Cyperus difformis*), hardstem bulrush (*Scirpus juncoides*), needle spikerush (*Eleocharis acicularis*), water nutgrass (*Cyperus serotinus*), water chestnut (*Eleocharis kuroguwai*)

### Pontederiaceous weeds:

monochoria (*Monochoria vaginalis*)

### Alismataceous weeds:

arrowhead (*Sagittaria pygmaea*), arrowhead (*Sagittaria trifoli*a), waterplantain (*Alisma canaliculatum*)

### Potamogetonaceous weeds:

roundleaf pondweed (*Potamogeton distinctus*)

### Umbelloferous weeds:

watercelery sp. (*Oenanthe javanica*)

Furthermore, some of the present compounds exhibit no significant phytotoxicity on transplanted paddy rice.

The present compounds can also attain the control of a wide variety of weeds which are growing or will grow in the orchards, grasslands, lawns, forests, waterways, canals, or other non-cultivated lands.

The present compounds also have herbicidal activity against various aquatic weeds, such as water hyacinth (*Eichhornia crassipes*), which are growing or will grow at the waterside such as waterways or canals.

The present compounds have substantially the same characteristics as those of the herbicidal compounds disclosed in the published specification of International Patent Application, WO95/34659. In the case where crops with tolerance imparted by introducing a herbicide tolerance gene described in the published specification are cultivated, the present compounds can be used at larger rates than those used when ordinary crops without tolerance are cultivated, which makes it possible to control other unfavorable weeds more effectively.

When the present compounds are used as the active ingredients of herbicides, they are usually mixed with solid or liquid carriers or diluents, surfactants, and other auxiliary agents to give emulsifiable concentrates, wettable powders, flowables, granules, concentrated emulsions, water-dispersible granules, or other formulations.

These formulations may contain any of the present compounds as an active ingredient at an amount of 0.001 to 80% by weight, preferably 0.005 to 70% by weight, based on the total weight of the formulation.

The solid carrier or diluent which can be used may include, for example, fine powders or granules of the following materials: mineral matters such as kaolin clay, attapulgite clay, bentonite, acid clay, pyrophyllite, talc, diatomaceous earth, and calcite; organic substances such as walnut shell powder; water-soluble organic substances such as urea; inorganic salts such as ammonium sulfate; and synthetic hydrated silicon oxide. The liquid carrier or diluent which can be used may include, for example, aromatic hydrocarbons such as methylnaphthalene, phenylxylylethane, and alkylbenzene (e.g., xylene); alcohols such as isopropanol, ethylene glycol, and 2-ethoxyethanol; esters such as phthalic acid dialkyl esters; ketones such as acetone, cyclohexanone, and isophorone; mineral oils such as machine oil; vegetable oils such as soybean oil and cottonseed oil; dimethylsulfoxide, N,N-dimethylformamide, acetonitrile, N-methylpyrrolidone, and water.

The surfactant used for emulsification, dispersing, or spreading may include surfactants of the anionic type, such as alkylsulfates, alkylsulfonates, alkylarylsulfonates, dialkylsulfosuccinates, and phosphates of polyoxyethylene alkyl aryl ethers; and surfactants of the nonionic type, such as polyoxyethylene alkyl ethers, polyoxyethylene alkyl aryl ethers, polyoxyethylene polyoxypropylene block copolymers, sorbitan fatty acid esters, and polyoxyethylene sorbitan fatty acid esters.

The auxiliary agent may include lignin sulfonates, alginates, polyvinyl alcohol, gum arabic, CMC (carboxymethylcellulose), and PAP (isopropyl acid phosphate).

The present compounds are usually formulated as described above and then used for pre- or post-emergence soil, foliar, or flooding treatment of weeds. The soil treatment may include soil surface treatment and soil incorporation. The foliar treatment may include application over the plants and directed application in which a chemical is applied only to weeds so as to keep off the crop plants.

The present compounds may often exhibit the enhancement of herbicidal activity when used in admixture with other herbicides. They can also be used in admixture with insecticides, acaricides, nematocides, fungicides, bactericides, plant growth regulators, fertilizers, and soil conditioners.

Examples of the herbicide which can be used in admixture with the present compounds are atrazin, cyanazine, dimethametryn, metribuzin, prometryn, simazine, simetryn, chlorotoluron, diuron, dymrone, fluometuron, isoproturon, linuron, methabenzthiazuron, bromoxynil, ioxynil, ethalfluralin, pendimethalin, trifluralin, acifluorfen, acifluorfen-sodium, bifenox, chlomethoxynil, fomesafen, lactofen, oxadiazon, oxadiargyl, oxyfluorfen, carfentrazon-ethyl, flumiclorac-pentyl, flumioxazine, fluthiacet-methyl, sulfentrazone, thidiazimin, azafenidin, pyraflufen-ethyl, difenzoquat, diquat, paraquat, 2,4-D, 2,4-DB, DCPA, MCPA, MCPB, clomeprop, clopyralid, dicamba, dithiopyr, fluroxypyr, mecoprop, naproanilide, phenothiol, quinclorac, triclopyr, thiazopyr, acetochlor, alachlor, butachlor, diethatyl-ethyl, metolachlor, pretilachlor, propachlor, bensulfuron-methyl, chlorsulfuron, chlorimuron-ethyl, halosulfuron-methyl, metsulfuron-methyl, nicosulfuron, primisulfuron-methyl, pyrazosulfuron-ethyl, sulfometuron-methyl, thifensulfuron-methyl, triasulfuron, tribenuron-methyl, oxasulfuron, azimsulfuron, cloransulam-methyl, cyclosulfamuron, flumetsulam, flupyrsulfuron, flazasulfuron, imazosulfuron, metosulam, diclosulam, prosulfuron, rimsulfuron triflusulfuron-methyl, ethoxysulfuron, sulfosulfuron, imazamethabenzmethyl, imazapyr, imazaquin, imazethapyr, imazameth, imazamox, bispyribac-sodium, pyriminobac-methyl, pyrithiobac-sodium, alloxydim-sodium, clethodim, sethoxydim, tralkoxydim, dichlofop-methyl, fenoxaprop-ethyl, fenoxaprop-p-ethyl, fluazifop-butyl, fluazifop-p-butyl, haloxyfop-methyl, quizalofop-p-ethyl, cyhalofop-butyl, clodinafop-propargyl, benzofenap, clomazone, diflufenican, norflurazon, pyrazolate, pyrazoxyfen, flurtamone, isoxaflutole, sulcotrione, glufosinate-ammonium, glyphosate, bentazon, benthiocarb, bromobutide, butamifos, butylate, dimepiperate, dimethenamid, DSMA, EPTC, esprocarb, isoxaben, mefenacet, molinate, MSMA, piperophos, pyributicarb, propanil, pyridate, triallate, cafenstrol, flupoxam, fluthiamide, diflufenzopyr, triaziflam, pentoxazone, epoprodam, metobenzuron, and oxaziclomefon.

The above compounds are described in the catalog of Farm Chemicals Handbook, 1995 (published by Meister Publishing Company); AG CHEM NEW COMPOUND REVIEW, VOL. 13, 1995 or VOL. 15, 1997 (published by AG CHEM INFORMATION SERVICES); "Josouzai Kenkyu Souran" (published by Hakuyu-sha); or HERBICIDE HANDBOOK, Seventh Edition (published by Weed Science Society of America).

The following will describe some particular examples of the above admixture, in which the present compounds are designated by their compound numbers shown in Tables 1 to 2 below.
1. A compound selected from the group consisting of the present compounds 1-54, 1-58, 1-66, 1-70, 1-98, 1-102, 1-194, 1-198, 1-258, 1-262, 1-266, 1-274, 1-306, 1-322, 2-2, and 2-10 is mixed with a compound selected from the group consisting of atrazin, cyanazine, bromoxynil, and bentazon at a weight ratio of 1 : 0.1 to 1000.
2. A compound selected from the group consisting of the present compounds 1-54, 1-58, 1-66, 1-70, 1-98, 1-102, 1-194, 1-198, 1-258, 1-262, 1-266, 1-274, 1-306, 1-322, 2-2, and 2-10 is mixed with a compound selected from the group consisting of clethodim, sethoxydim, dichlofop-methyl, quizalofop-p-ethyl, lactofen, acifluorfen, acifluorfen-sodium, fomesafen, flumiclorac-pentyl, and dicamba at a weight ratio of 1 : 0.01 to 500.
3. A compound selected from the group consisting of the present compounds 1-54, 1-58, 1-66, 1-70, 1-98, 1-102, 1-194, 1-198, 1-258, 1-262, 1-266, 1-274, 1-306, 1-322, 2-2, and 2-10 is mixed with a compound selected from the group consisting of nicosulfuron, primisulfuron, prosulfuron, chlorimuron-ethyl, thifensulfuron-methyl, and imazamox at a weight ratio of 1 : 0.01 to 100.
4. A compound selected from the group consisting of the present compounds 1-54, 1-58, 1-66, 1-70, 1-98, 1-102, 1-194, 1-198, 1-258, 1-262, 1-266, 1-274, 1-306, 1-322, 2-2, and 2-10 is mixed with a compound selected from the group consisting of isoproturon and chlorotoluron at a weight ratio of 1 : 0.1 to 1000.
5. A compound selected from the group consisting of the present compounds 1-54, 1-58, 1-66, 1-70, 1-98, 1-102, 1-194, 1-198, 1-258, 1-262, 1-266, 1-274, 1-306, 1-322, 2-2, and 2-10 is mixed with a compound selected from the group consisting of mecoprop, fluroxypyr, and ioxynil at a weight ratio of 1 : 0.05 to 500.
6. A compound selected from the group consisting of the present compounds 1-54, 1-58, 1-66, 1-70, 1-98, 1-102, 1-194, 1-198, 1-258, 1-262, 1-266, 1-274, 1-306, 1-322, 2-2, and 2-10 is mixed with a compound selected from the group consisting of diflufenican, metsulfuron-methyl, fenoxapropethyl, and clodinafop-propargyl at a weight ratio of 1 : 0.001 to 100.

When the present compounds are used as the active ingredients of herbicides, the application amount, although it may vary with the weather conditions, formulation types, application times, application methods, soil conditions, crops to be protected, weeds to be controlled, and other factors, is usually in the range of 0.01 to 10,000 g, preferably 1 to 8000 g, per hectare. In the case of emulsifiable concentrates, wettable powders, flowables, concentrated emulsions, water-dispersible granules, or other similar formulations, they are usually applied after diluted in their prescribed amounts with water (if necessary, containing an adjuvant such as a spreading agent) at a ratio of 10 to 1000 liters per hectare. In the case of granules or some types of flowables, they are usually applied as such without any dilution.

The adjuvant which can be used, if necessary, may include, in addition to the surfactants as described above, polyoxyethylene resin acids (esters), lignin sulfonates, abietates, dinaphthylmethanedisulfonates, crop oil concentrates, and vegetable oils such as soybean oil, corn oil, cottonseed oil, and sunflower oil.

The present compounds can also be used as the active ingredients of harvesting aids such as defoliants and desiccants for cotton (*Gossipyum* spp.), and desiccants for potato (*Solanum tuberosum*). In these cases, the present compounds are usually formulated in the same manner as the case where they are used as the active ingredients of herbicides, and used alone or in admixture with other harvesting aids for foliar treatment before the harvesting of crops.

### Examples

The present invention will be further illustrated by the following production examples, formulation examples, and test examples; however, the present invention is not limited to these examples.

The following will describe production examples for the present compounds and their production intermediates.

### Production Example 1 (Production of compound [XIII] shown in the following scheme 7)

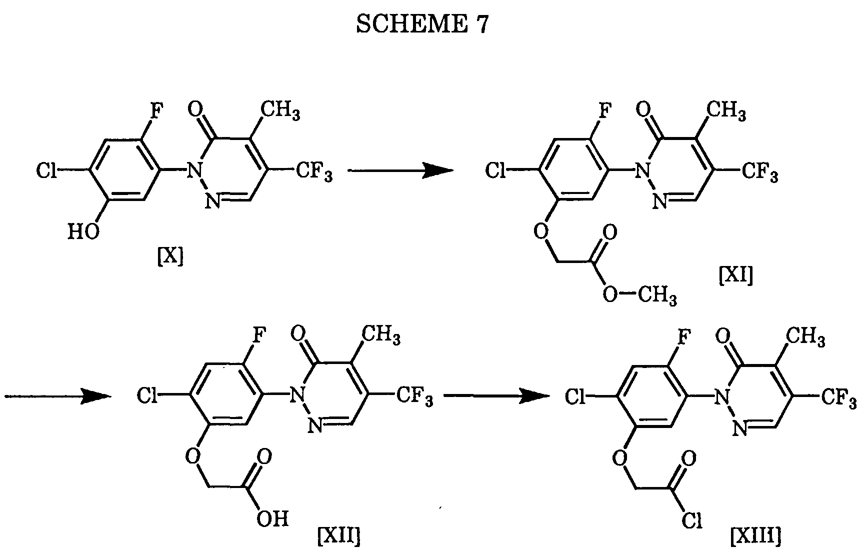

To a solution of 1.0 g of compound [X] (produced in Reference Production Example described below) in 10 ml of N,N-dimethylformamide (hereinafter referred to as DMF) were added 0.5 g of potassium carbonate and 0.5 g of methyl bromoacetate. After stirring at room temperature for 1 hour, water was added to the mixture, which was then extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 1.1 g of compound [XI], m.p., 80.4°C.

To a solution of 1.1 g of compound [XI] in 15 ml of 1,4-dioxane was added 10 ml of concentrated hydrochloric acid. After stirring at 50°C for 5.5 hours, water was added to the mixture, which was extracted with ethyl acetate. The organic layer was washed with saturated aqueous sodium chloride solution, and then dried over anhydrous magnesium sulfate. Toluene was added, and the solvent was evaporated under reduced pressure, which afforded 1.1 g of crude compound [XII], m.p., 182.2°C.

To a solution of 3.0 g of crude compound [XII] thus obtained in 30 ml of THF was added 2.2 g of thionyl chloride. After heating under reflux for 4.5 hours, the solvent was evaporated under reduced pressure, which afforded 3.1 g of crude compound [XIII].

### Production Example 2 (Production of compound [XVI] shown in the following scheme 8)

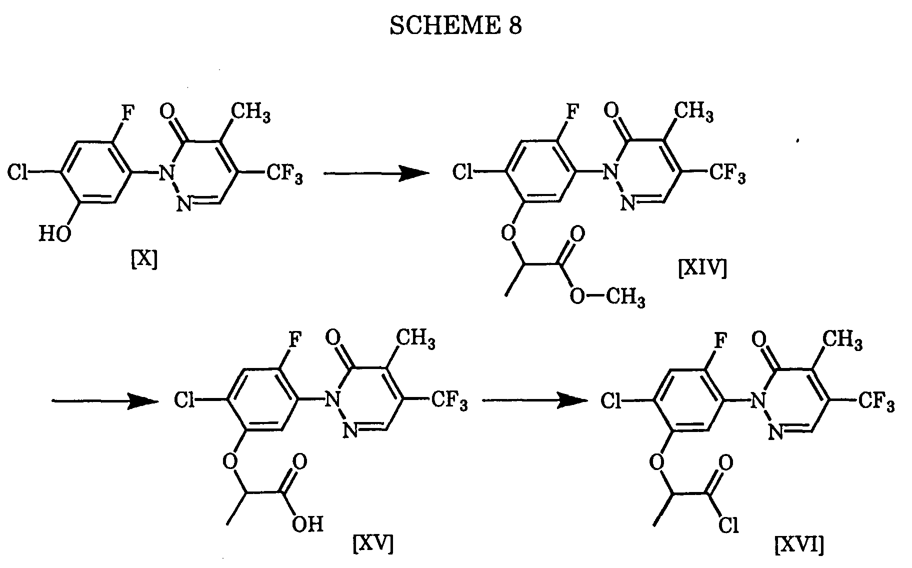

To a solution of 1.0 g of compound [X] in 10 ml of DMF were added 0.5 g of potassium carbonate and 0.6 g of methyl 2-bromopropionate. After stirring at room temperature for 1.5 hours, water was added to the mixture, which was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 1.2 g of compound [XIV].
¹H-NMR (250 MHz, CDCl₃, TMS, δ (ppm)): 1.68 (3H, d, J = 7 Hz), 2.43 (3H, q, J = 2 Hz), 3.76 (3H, s), 4.73 (1H, q, J = 7 Hz), 6.98 (1H, d, J = 7 Hz), 7.32 (1H, d, J = 8 Hz), 7.99 (1H, s).

To a solution of 1.2 g of compound [XIV] thus obtained in 15 ml of 1,4-dioxane was added 10 ml of concentrated hydrochloric acid. After stirring at 50°C for 5.5 hours, water was added to the mixture, which was extracted with ethyl acetate. The organic layer was washed with saturated aqueous sodium chloride solution, and then dried over anhydrous magnesium sulfate. Toluene was added, and the solvent was evaporated under reduced pressure, which afforded 1.2 g of crude compound [XV].
¹H-NMR (250 MHz, CDCl₃, TMS, δ (ppm)): 1.70 (3H, d, J = 6.8 Hz), 2.42 (3H, q, J = 1.8 Hz), 4.74 (1H, q, J = 6.8 Hz), 6.02 (1H, br), 7.02 (1H, d, J = 6.4 Hz), 7.33 (1H, d, J = 9.2 Hz), 8.00 (1H, s).

To a solution of 5.0 g of crude compound [XV] thus obtained in 45 ml of THF was added under ice cooling a mixed solution of 3.6 g of thionyl chloride and 30 ml of THF. After stirring at room temperature for 0.5 hours, the mixture was heated under reflux for 4.0 hours. The solvent was evaporated under reduced pressure, which afforded 5.0 g of crude compound [XVI].

### Production Example 3 (Production of the present compound 1-66)

To a solution of 0.5 g of compound [X] obtained in 5 ml of DMF were added 0.2 g of potassium carbonate and 0.2 g of allyl chloroacetate. After stirring at room temperature for 2.0 hours and then at 60°C for 0.5 hours, water was added to the mixture, which was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.4 g of the present compound 1-66.
¹H-NMR (300 MHz, CDCl₃, TMS, δ (ppm)): 2.42 (3H, q, J = 1.9 Hz), 4.67-4.72 (4H, m), 5.23-5.37 (2H, m), 5.84-5.98 (1H, m), 7.00 (1H, d, J = 6.3 Hz), 7.33 (1H, d, J = 9.2 Hz), 7.99 (1H, s).

### Production Example 4 (Production of the present compound 1-58)

To a solution of 0.5 g of compound [X] in 5 ml of DMF were added 0.2 g of potassium carbonate and 0.2 g of vinyl chloroacetate. After stirring at room temperature for 5.0 hours, water was added to the mixture, which was extracted with ethyl acetate. The organic layer was washed with aqueous hydrochloric acid solution and saturated aqueous sodium chloride solution in this order. The solvent was evaporated under reduced pressure, and the residue was then subjected to silica gel column chromatography, which afforded 0.2 g of the present compound 1-58.
¹H-NMR (300 MHz, CDCl₃, TMS, δ (ppm)): 2.42 (3H, q, J = 1.8 Hz), 4.68-4.71 (1H, m), 4.77 (2H, s), 4.94-5.01 (1H, m), 7.03 (1H, d, J = 6.3 Hz), 7.26-7.31 (1H, m), 7.34 (1H, d, J = 9.0 Hz), 7.99 (1H, s).

### Production Example 5 (Production of the present compound 1-54)

To a solution of 0.5 g of compound [X] in 5 ml of DMF were added 0.2 g of potassium carbonate and 0.5 g of 2-bromo-1-phenyl-1-propanone. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.6 g of the present compound 1-54.
¹H-NMR (250 MHz, CDCl₃, TMS, δ (ppm)): 1.78 (3H, d, J = 6.9 Hz), 2.40 (3H, q, J = 1.9 Hz), 5.39 (1H, q, J = 6.9 Hz), 6.93 (1H, d, J = 6.4 Hz), 7.30 (1H, d, J = 9.2 Hz), 7.43-7.63 (3H, m), 7.94 (1H, s), 8.04-8.08 (2H, m).

### Production Example 6 (Production of the present compound 1-258)

To a solution of 0.3 g of phenol in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.3 g of the present compound 1-258.
¹H-NMR (250 MHz, CDCl₃, TMS, δ (ppm)): 2.43 (3H, q, J = 1.9 Hz), 4.94 (2H, s), 7.10-7.43 (7H, m), 7.99 (1H, s).

### Production Example 7 (Production of the present compound 1-194)

To a solution of 0.4 g of benzyl alcohol in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography. The resulting crystals were washed with hexane, which afforded 0.4 g of the present compound 1-194.
¹H-NMR (300 MHz, CDCl₃, TMS, δ (ppm)): 2.43 (3H, q, J = 2.0 Hz), 4.72 (2H, s), 5.23 (2H, s), 6.95 (1H, d, J = 6.3 Hz), 7.30-7.38 (6H, m), 7.96 (1H, s).

### Production Example 8 (Production of the present compound 1-98)

To a solution of 0.3 g of 2-butynyl alcohol in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.5 g of the present compound 1-98.
¹H-NMR (300 MHz, CDCl₃, TMS, δ (ppm)): 1.84 (3H, t, J = 2.4 Hz), 2.43 (3H, q, J = 1.7 Hz), 4.71 (2H, s), 4.76 (2H, q, J = 2.4 Hz), 7.01 (1H, d, J = 6.3 Hz), 7.33 (1H, d, J = 9.2 Hz), 7.99 (1H, s).

### Production Example 9 (Production of the present compound 1-266)

To a solution of 0.2 g of azetidine in 5 ml of THF was added 0.5 ml of pyridine and added dropwise 5 ml of a THF solution containing 1.2 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.5 g of the present compound 1-266.
¹H-NMR (250 MHz, CDCl₃, TMS, δ (ppm)): 2.33 (2H, tt, J = 7.8 Hz, J = 7.8 Hz), 2.43 (3H, q, J = 1.9 Hz), 4.11 (2H, t, J = 7.8 Hz), 4.42 (2H, t, J = 7.8 Hz), 4.61 (2H, s), 6.99 (1H, d, J = 6.2 Hz), 7.34 (1H, d, J = 9.0 Hz), 8.00 (1H, s).

### Production Example 10 (Production of the present compound 1-274)

To a solution of 0.4 g of thiomorpholine in 5 ml of THF was added 0.5 ml of pyridine and added dropwise 5 ml of a THF solution containing 1.2 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.4 g of the present compound 1-274.
¹H-NMR (300 MHz, CDCl₃, TMS, δ (ppm)): 2.43 (3H, q, J = 1.8 Hz), 2.59-2.69 (4H, m), 3.84-3.89 (4H, m), 4.74 (2H, s), 7.12 (1H, d, J = 6.3 Hz), 7.33 (1H, d, J = 9.2 Hz), 7.99 (1H, s).

### Production Example 11 (Production of the present compound 1-322)

To a solution of 0.3 g of 2-aminoethanol in 5 ml of THF was added 0.5 ml of pyridine and added dropwise 7.5 ml of a THF solution containing 2.0 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.7 g of the present compound 1-322.
¹H-NMR (300 MHz, CDCl₃, TMS, δ (ppm)): 2.43 (3H, q, J = 1.9 Hz), 3.50-3.57 (2H, m), 3.76 (2H, t, J = 4.8 Hz), 4.57 (1H, s), 6.97 (1H, d, J = 6.2 Hz), 7.06 (1H, br), 7.37 (1H, d, J = 8.9 Hz), 8.01 (1H, s).

### Production Example 12 (Production of the present compound 1-306)

To a solution of 0.4 g of the present compound 1-322 (produced in Production Example 11 described above) in 4 ml of acetic anhydride was added 2 ml of pyridine. After stirring at room temperature for 0.5 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.4 g of the present compound 1-306.
¹H-NMR (300 MHz, CDCl₃, TMS, δ (ppm)): 2.08 (3H, s), 2.44 (3H, q, J = 1.8 Hz), 3.60-3.71 (2H, m), 4.22 (2H, t, J = 5.3 Hz), 4.53 (2H, s), 6.98 (1H, d, J = 6.2 Hz), 7.03 (1H, br), 7.37 (1H, d, J = 8.9 Hz), 8.00 (1H, s).

### Production Example 13 (Production of the present compound 2-2)

To a solution of 0.6 g of 1,4-anhydroerythritol in 50 ml of THF was added 1.0 ml of pyridine and then added dropwise under ice cooling 30 ml of a THF solution containing 2.6 mmol of compound [XIII]. After stirring at 0°C for 2.0 hours, the mixture was left stand at room temperature overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with toluene, which afforded 0.5 g of the present compound 2-2.
¹H-NMR (300 MHz, CDCl₃, TMS, δ (ppm)): 2.42 (3H, q, J = 1.8 Hz), 2.62 (1H, br), 3.57-3.63 (1H, m), 3.83-3.90 (1H, m), 3.92-4.00 (1H, m), 4.02-4.10 (1H, m), 4.45-4.54 (1H, m), 4.81 (2H, s), 5.21-5.28 (1H, m), 7.03 (1H, d, J = 6.3 Hz), 7.36 (1H, d, J = 9.0 Hz), 8.01 (1H, s).

### Production Example 14 (Production of the present compound 2-10)

To a solution of 0.5 g of the present compound 2-2 (produced in Production Example 13 described above) in 5 ml of acetic anhydride was added 5 ml of pyridine. After stirring at room temperature for 2.0 hours the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.5 g of the present compound 2-10.
¹H-NMR (250 MHz, CDCl₃, TMS, δ (ppm)): 2.03 (3H, s), 2.43 (3H, q, J = 1.8 Hz), 3.75-3.90 (2H, m), 4.02-4.14 (2H, m), 4.72 (2H, d, J = 2.5 Hz), 5.28-5.38 (1H, m), 5.41-5.50 (1H, m), 7.03 (1H, d, J = 6.3 Hz), 7.34 (1H, d, J = 9.2 Hz), 7.99 (1H, s).

### Production Example 15 (Production of the present compound 1-262)

To a solution of 0.3 g of phenol in 5 ml of THF was added 0.5 ml of pyridine and added dropwise 5 ml of a THF solution containing 1.2 mmol of compound [XVI]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.3 g of the present compound 1-262.
¹H-NMR (300 MHz, CDCl₃, TMS, δ (ppm)): 1.85 (3H, d, J = 6.8 Hz), 2.43 (3H, q, J = 2.0 Hz), 4.93 (1H, q, J = 6.8 Hz), 7.05-7.41 (7H, m), 7.97 (1H, s).

### Production Example 16 (Production of the present compound 1-198)

To a solution of 0.4 g of benzyl alcohol in 5 ml of THF was added 0.5 ml of pyridine and added dropwise 5 ml of a THF solution containing 1.2 mmol of compound [XVI]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.4 g of the present compound 1-198.
¹H-NMR (300 MHz, CDCl₃, TMS, δ (ppm)): 1.69 (3H, d, J = 6.7 Hz), 2.42 (3H, q, J = 1.9 Hz), 4.74 (1H, q, J = 6.7 Hz), 5.18 (2H, s), 6.94 (1H, d, J = 6.4 Hz), 7.27-7.38 (6H, m), 7.94 (1H, s).

### Production Example 17 (Production of the present compound 1-102)

To a solution of 0.2 g of 2-butynyl alcohol in 5 ml of THF was added 0.5 ml of pyridine and added dropwise 5 ml of a THF solution containing 1.2 mmol of compound [XVI]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.3 g of the present compound 1-102.
¹H-NMR (300 MHz, CDCl₃, TMS, δ (ppm)): 1.69 (3H, d, J = 6.7 Hz), 1.82 (3H, t, J = 2.3 Hz), 2.42 (3H, q, J = 2.0 Hz), 4.69-4.77 (3H, m), 7.03 (1H, d, J = 6.4 Hz), 7.31 (1H, d, J = 9.2 Hz), 7.98 (1H, s).

### Production Example 18 (Production of the present compound 1-70)

To a solution of 0.2 g of allyl alcohol in 5 ml of THF was added 0.5 ml of pyridine and added dropwise 5 ml of a THF solution containing 1.2 mmol of compound [XVI]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.3 g of the present compound 1-70.
¹H-NMR (300 MHz, CDCl₃, TMS, δ (ppm)): 1.69 (3H, d, J = 6.9 Hz), 2.42 (3H, q, J = 1.9 Hz), 4.63-4.67 (2H, m), 4.73 (1H, q, J = 6.9 Hz), 5.20-5.34 (2H, m), 5.80-5.95 (1H, m), 7.01 (1H, d, J = 6.4 Hz), 7.32 (1H, d, J = 9.3 Hz),7.97 (1H, s).

### Production Example 19 (Production of the present compound 1-250)

To a solution of 0.4 g of α-phenetyl alcohol in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.5 g of the present compound 1-250.
¹H-NMR (250 MHz, CDCl₃, TMS, δ (ppm)): 1.56 (3H, d, J = 6.6 Hz), 2.42 (3H, q, J = 1.9 Hz), 4.65-4.71 (2H, m), 5.99 (1H, q, J = 6.6 Hz), 6.91 (1H, d, J = 6.3 Hz), 7.15-7.34 (6H, m), 7.95 (1H, s).

### Production Example 20 (Production of the present compound 1-234)

To a solution of 0.4 g of 4-methoxybenzyl alcohol in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.5 g of the present compound 1-234.
¹H-NMR (300 MHz, CDCl₃, TMS, δ (ppm)): 2.43 (3H, q, J = 1.9 Hz), 3.80 (3H, s), 4.69 (2H, s), 5.16 (2H, s), 6.83-6.95 (3H, m), 7.26-7.33 (3H, m), 7.97 (1H, s).

### Production Example 21 (Production of the present compound 1-210)

To a solution of 0.4 g of 4-nitrobenzyl alcohol in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.5 g of the present compound 1-210.
¹H-NMR (300 MHz, CDCl₃, TMS, δ (ppm)): 2.43 (3H, q, J = 1.8 Hz), 4.78 (2H, s), 5.32 (2H, s), 6.99 (1H, d, J = 6.3 Hz), 7.34 (1H, d, J = 9.1 Hz), 7.47-7.53 (2H, m), 7.98 (1H, s), 8.19-8.24 (2H, m).

### Production Example 22 (Production of the present compound 1-202)

To a solution of 0.5 g of 4-trifluoromethylbenzyl alcohol in 5 ml of THF was added 0.5 ml of pyridine and added dropwise 5 ml of a THF solution containing 1.3 mmol of compound [XIII]. After stirring at room temperature for 2.0 hours, water was added to the mixture, which was left stand overnight and then extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, the residue was then subjected to silica gel column chromatography, and the resulting crystals were washed with hexane, which afforded 0.6 g of the present compound 1-202.
¹H-NMR (300 MHz, CDCl₃, TMS, δ (ppm)): 2.43 (3H, q, J = 1.8 Hz), 4.75 (2H, s), 5.28 (2H, s), 6.98 (1H, d, J = 6.2 Hz), 7.34 (1H, d, J = 9.1 Hz), 7.44 (2H, d, J = 8.1 Hz), 7.61 (2H, d, J = 8.1 Hz), 7.97 (1H, s).

### Production Example 23 (Production of the present compound 1-338)

To a solution of 0.3 g of benzylamine in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.5 g of the present compound 1-338.
¹H-NMR (300 MHz, CDCl₃, TMS, δ (ppm)): 2.44 (3H, q, J = 1.7 Hz), 4.56-4.59 (4H, m), 6.98-7.08 (2H, m), 7.29-7.39 (6H, m), 8.00 (1H, s).

### Production Example 24 (Production of the present compound 1-354)

To a solution of 0.3 g of aniline in 5 ml of THF was added 0.5 ml of pyridine and added dropwise 5 ml of a THF solution containing 1.3 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane, which afforded 0.5 g of the present compound 1-354.
¹H-NMR (300 MHz, CDCl₃, TMS, δ (ppm)): 2.44 (3H, q, J = 1.7 Hz), 4.64 (2H, s), 7.06 (1H, d, J = 6.2 Hz), 7.15-7.21 (1H, m), 7.34-7.42 (3H, m), 7.59-7.63 (2H, m), 8.01 (1H, s), 8.51 (1H, br).

### Production Example 25 (Production of the present compounds 2-6 and 2-14)

To a solution of 2.0 g of 1,4-anhydroerythritol in 40 ml of THF was added 1.0 ml of pyridine and added dropwise under ice cooling 30 ml of a THF solution containing 2.1 mmol of compound [XVI]. After stirring at 0°C for 1.0 hour, the mixture was left stand at room temperature overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the residue was then subjected to silica gel column chromatography, which afforded 0.3 g of the present compound 2-6, m.p. 120.6°C.

To a solution of 0.3 g of the present compound 2-6 thus obtained in 5 ml of acetic anhydride was added 5 ml of pyridine. After stirring at room temperature for 2.0 hours, the mixture was left stand overnight. Toluene was added to the reaction mixture. The solvent was evaporated under reduced pressure, and the residue was then subjected to silica gel column chromatography, which afforded 0.3 g of the present compound 2-14.
¹H-NMR (250 MHz, CDCl₃, TMS, δ (ppm)): 1.68-1.72 (3H, m), 1.99-2.01 (3H, m), 2.42 (3H, q, J = 1.8 Hz), 3.69-4.11 (4H, m), 4.69-4.82 (1H, m), 5.25-5.45 (2H, m), 7.01-7.08 (1H, m), 7.33 (1H, d, J = 9.1 Hz), 7.98 (1H, s).

### Production Example 26 (Production of the present compound 1-362)

To a solution of 0.2 g of phenylhydrazine in 5 ml of THF was added 0.5 ml of pyridine and added dropwise 5 ml of a THF solution containing 0.9 mmol of compound [XIII]. After stirring at room temperature for 2.0 hours, the mixture was left stand overnight, to which water was added, and the mixture was extracted with ethyl acetate. The organic layer was washed with aqueous hydrochloric acid solution and saturated aqueous sodium chloride solution in this order, and then dried over anhydrous magnesium sulfate. The solvent was evaporated under reduced pressure, and the resulting crystals were then washed with hexane and toluene in this order, which afforded 0.2 g of the present compound 1-362.
¹H-NMR (300 MHz, CDCl₃, TMS, δ (ppm)): 2.44 (3H, q, J = 1.7 Hz), 4.69 (2H, s), 6.87 (2H, d, J = 7.8 Hz), 6.94 (1H, t, J = 7.4 Hz), 7.03 (1H, d, J = 6.2 Hz), 7.23-7.29 (2H, m), 7.40 (1H, d, J = 8.9 Hz), 8.01 (1H, s), 8.38 (1H, br).

Some examples of the present compounds are shown together with their compound numbers in Tables 1 and 2.

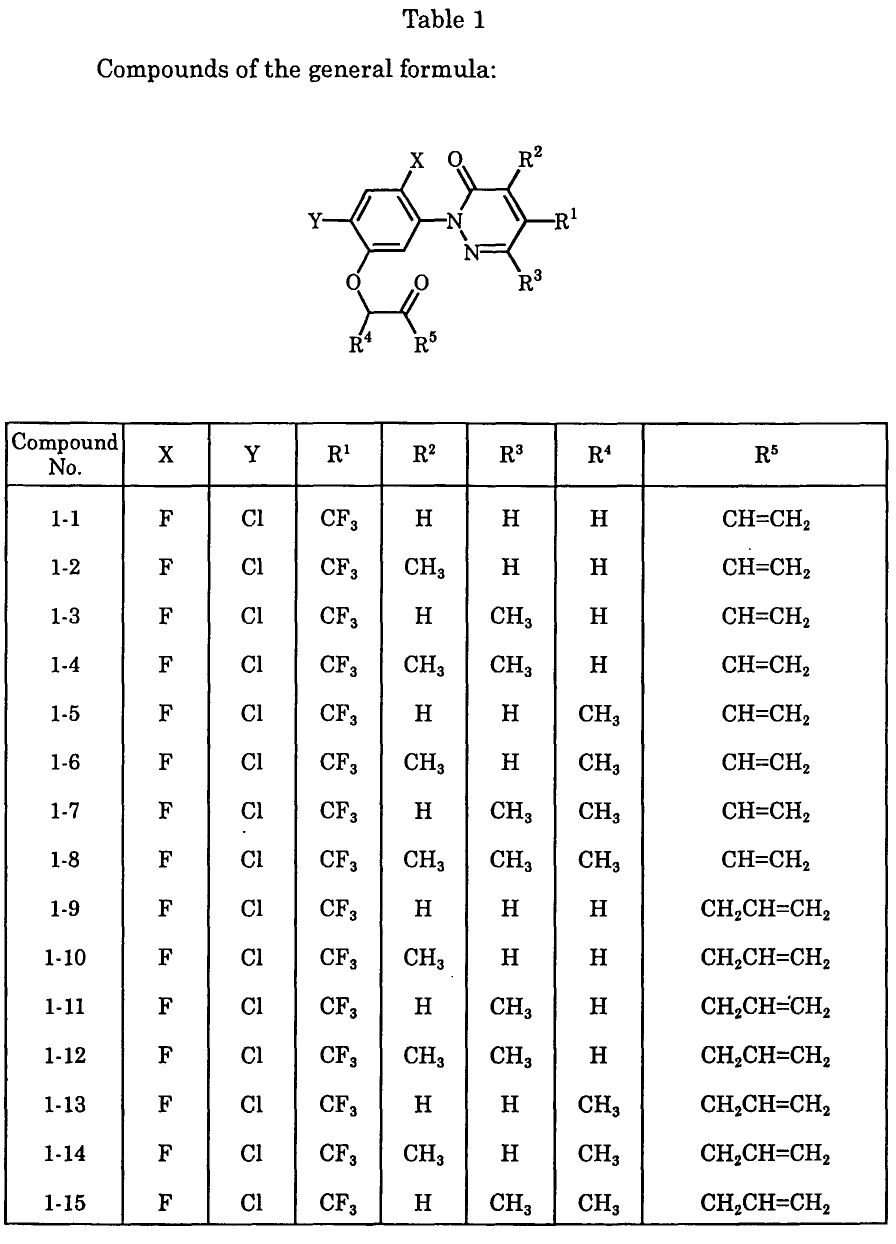

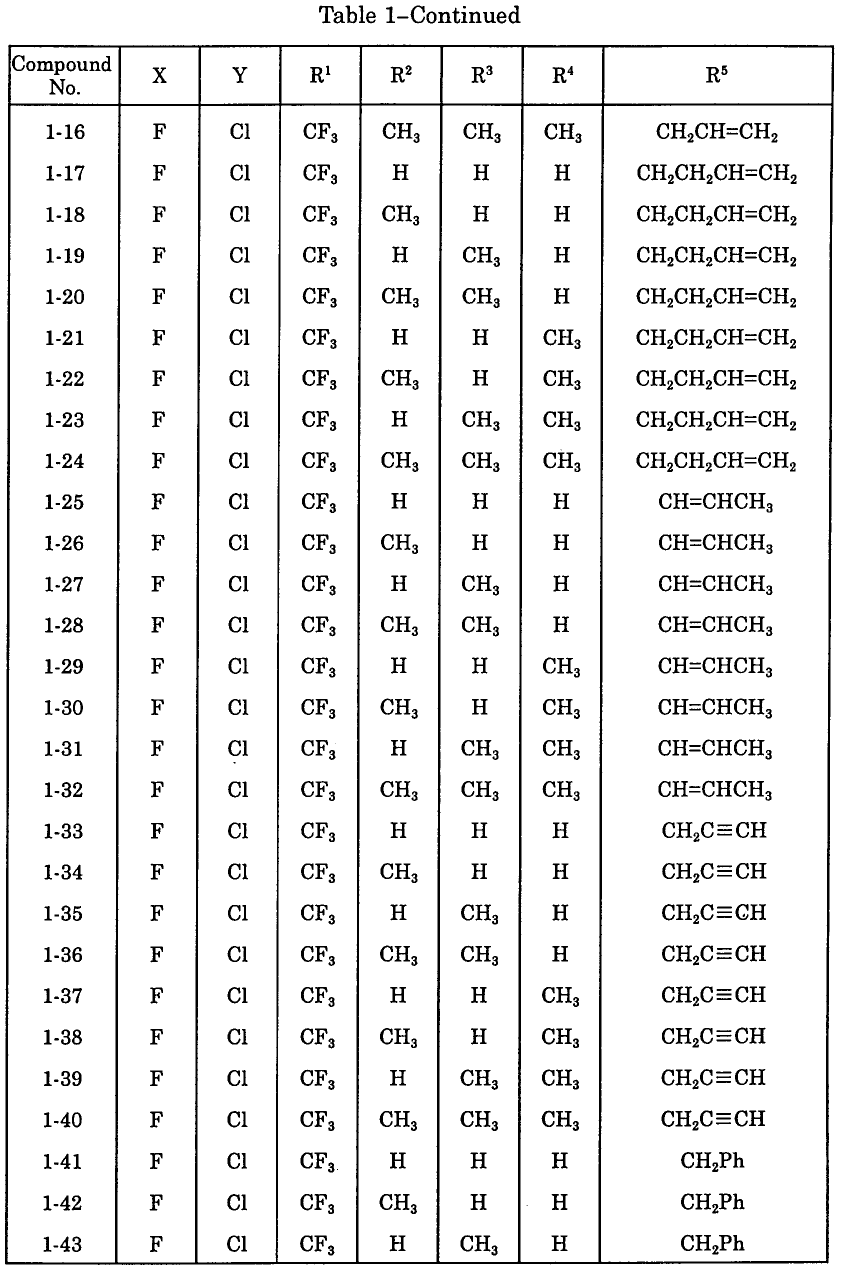

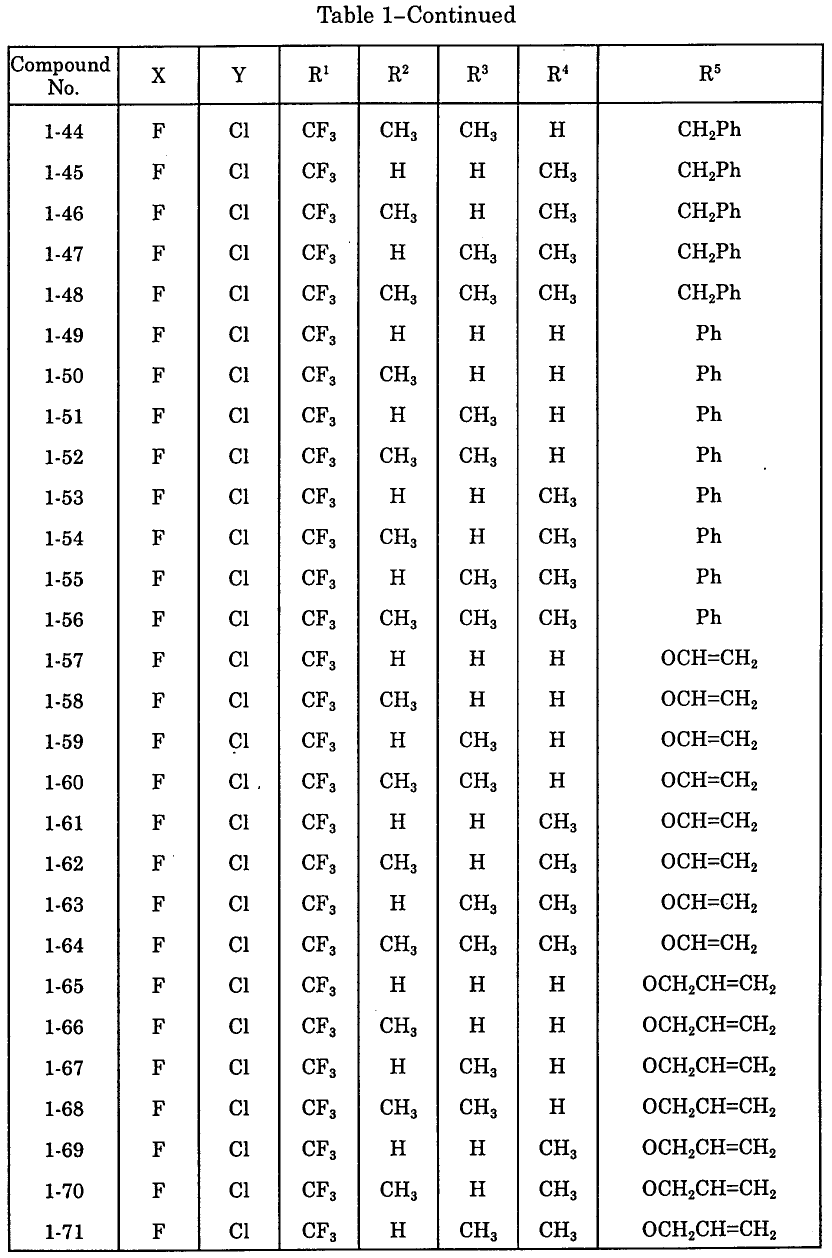

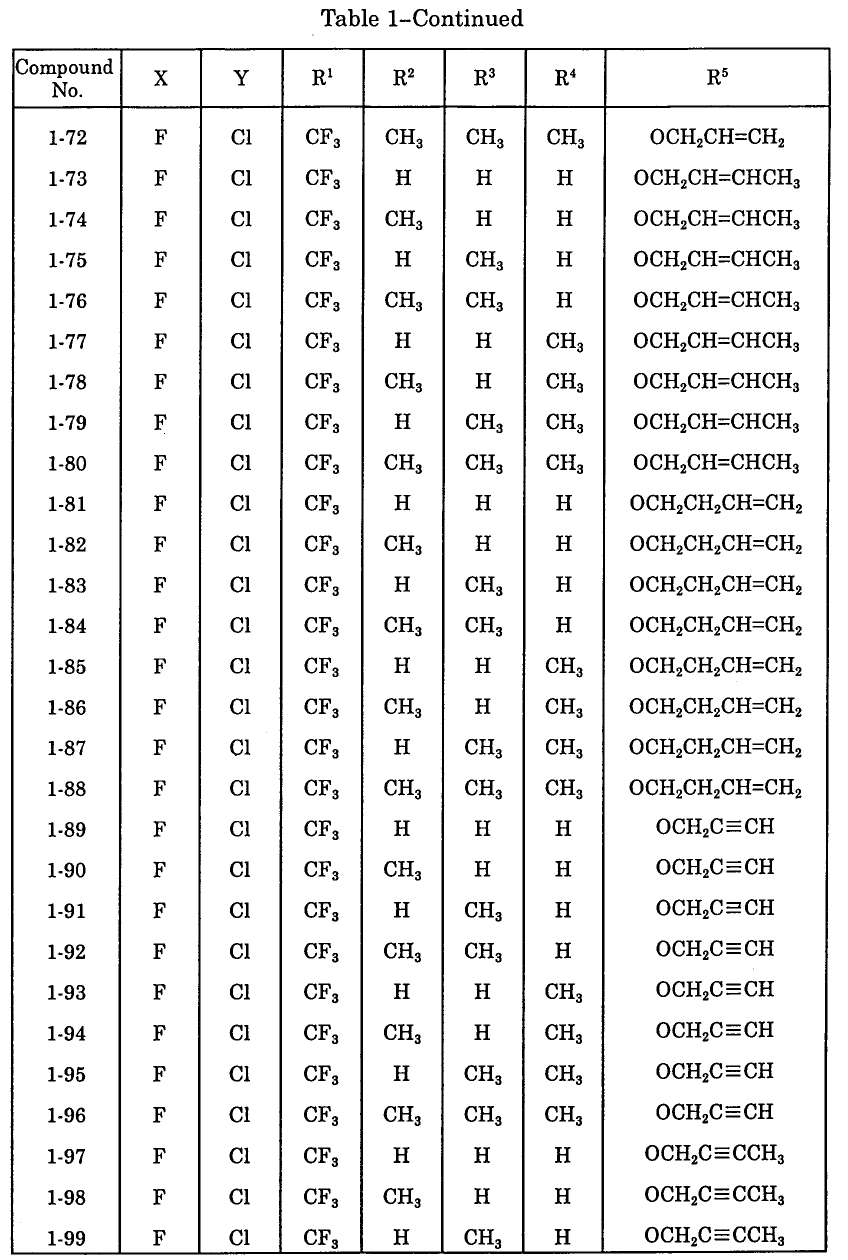

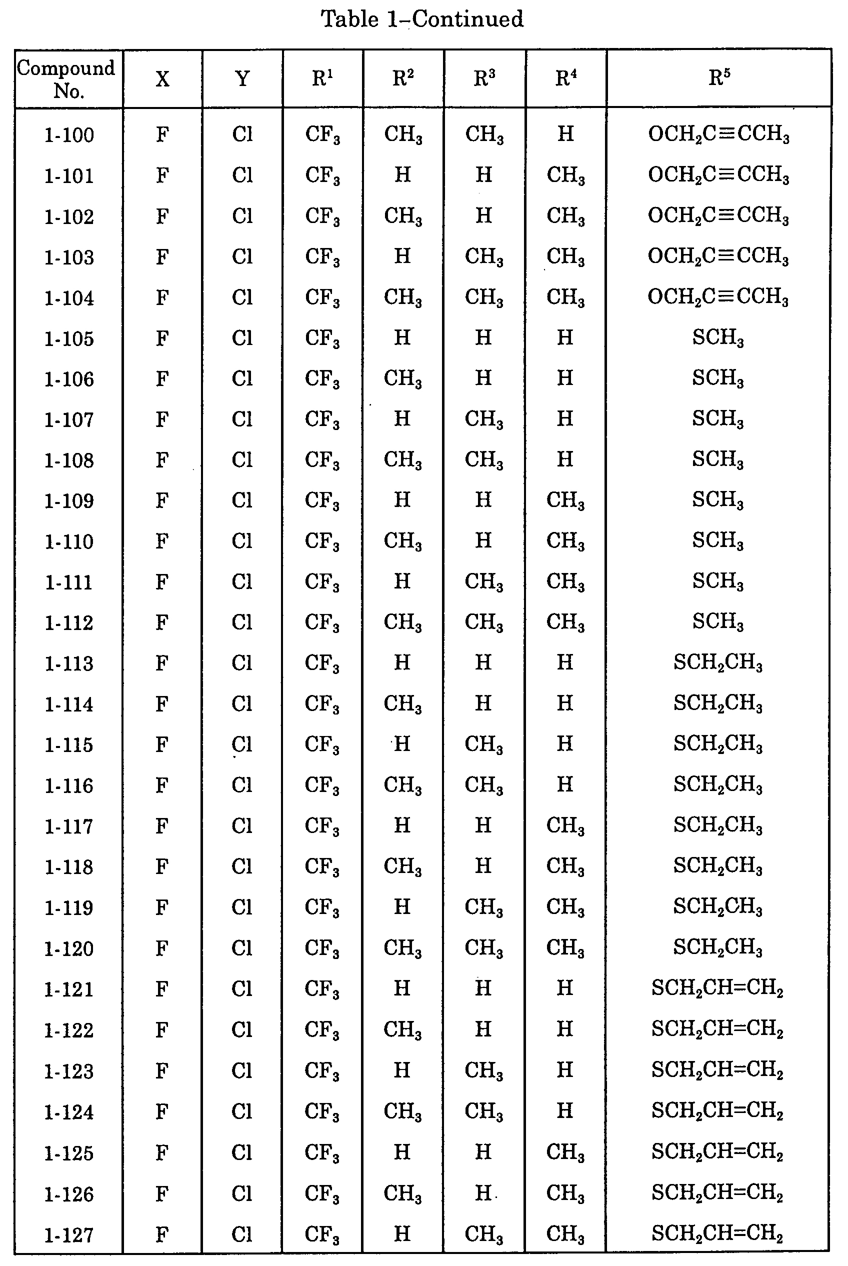

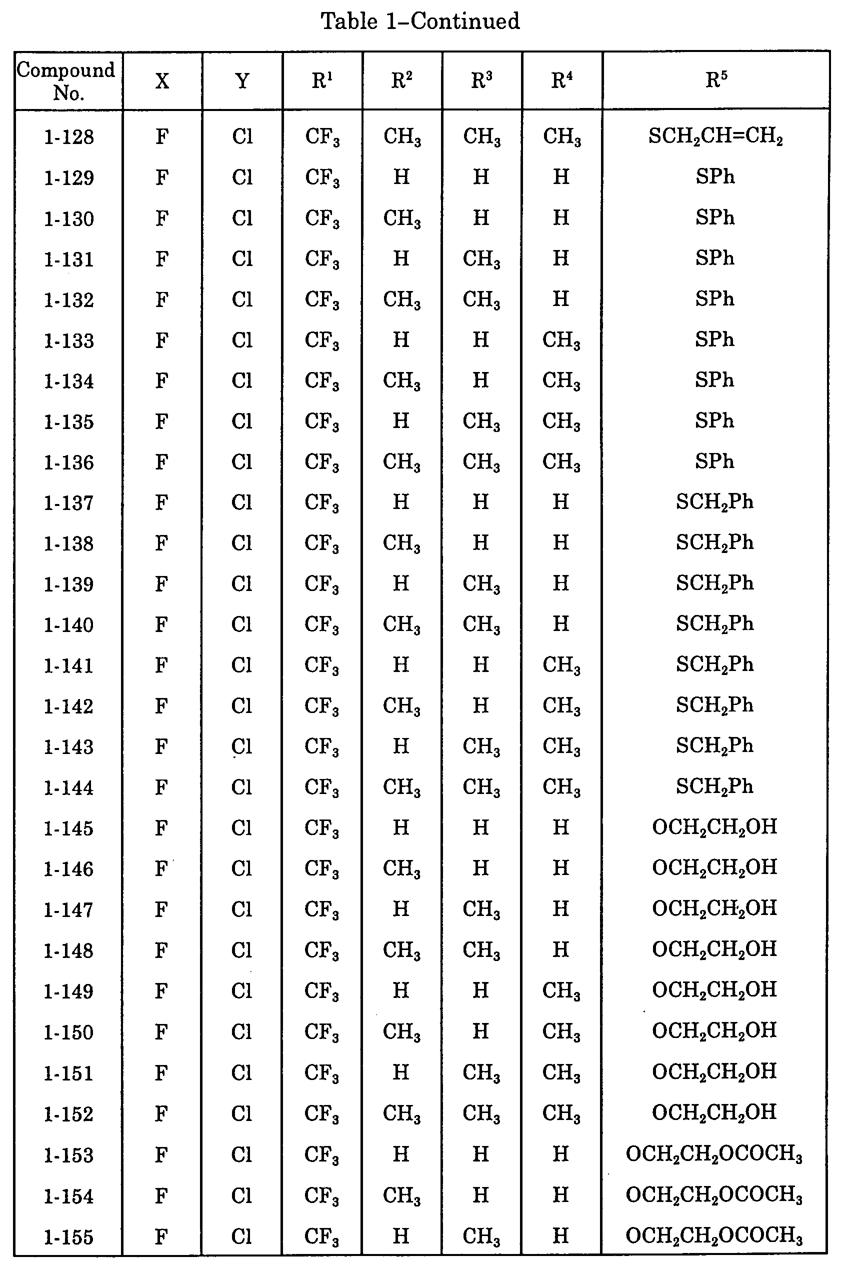

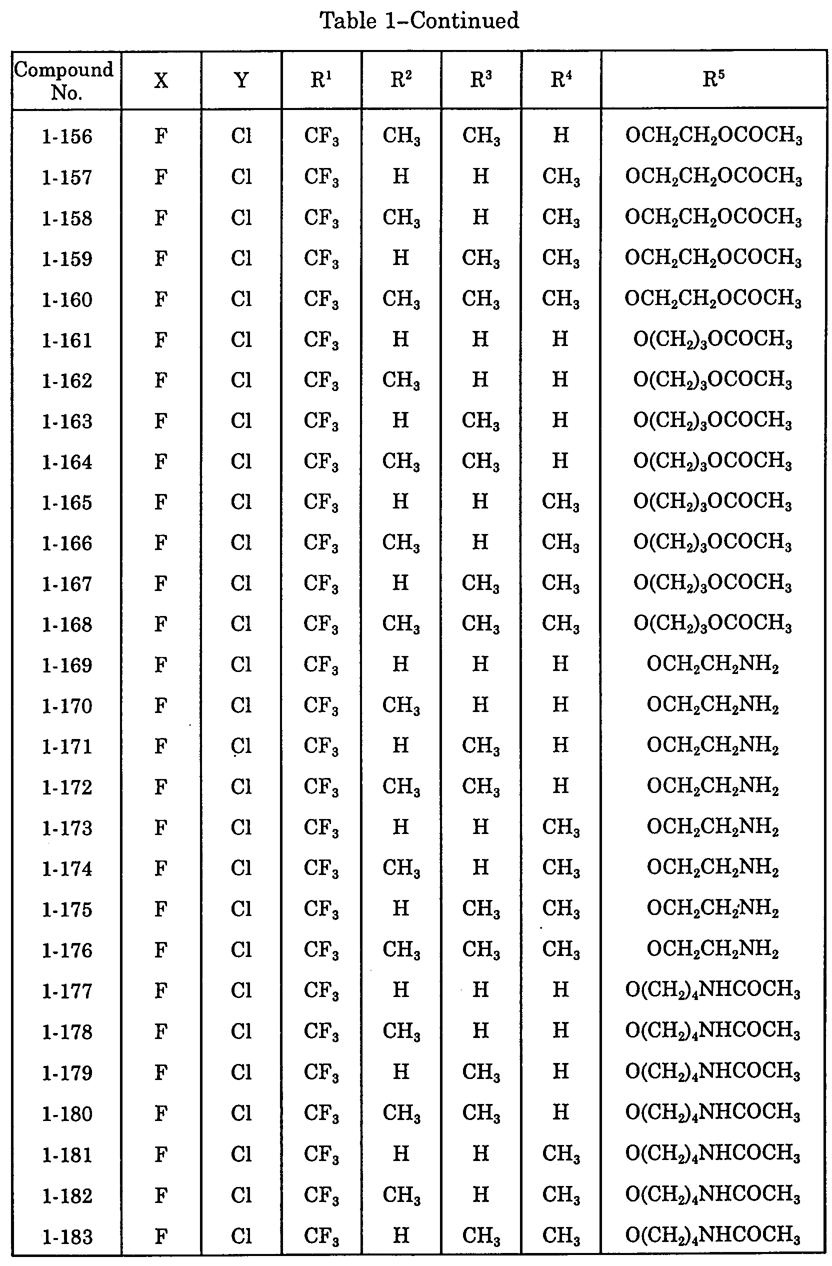

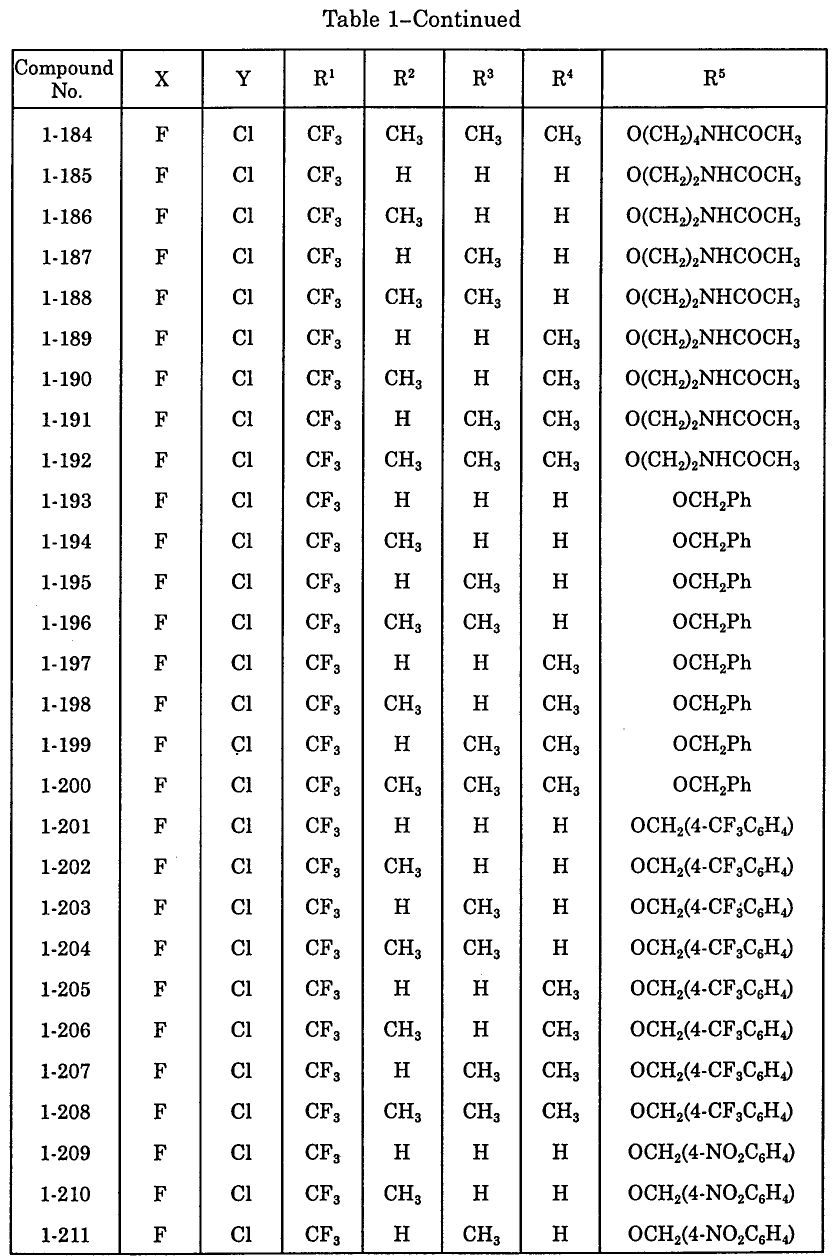

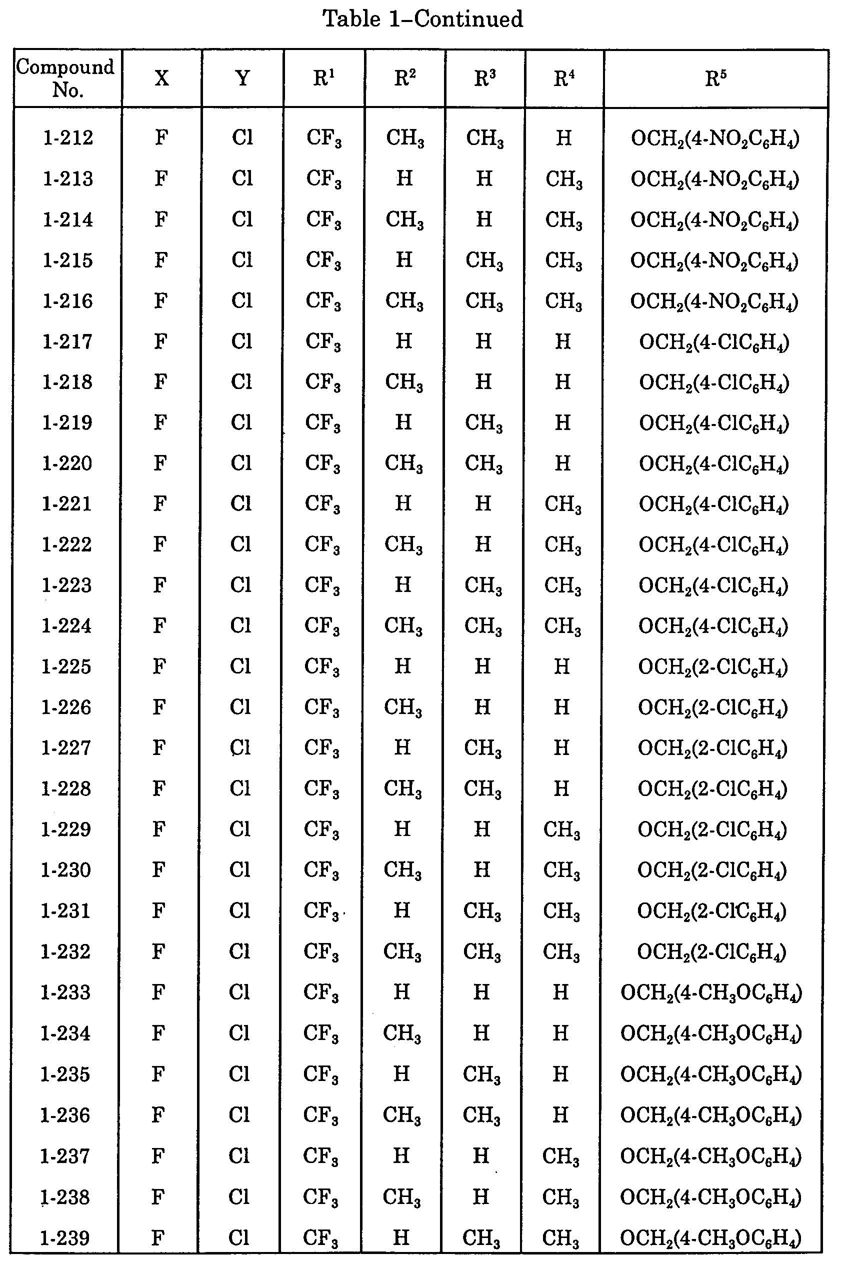

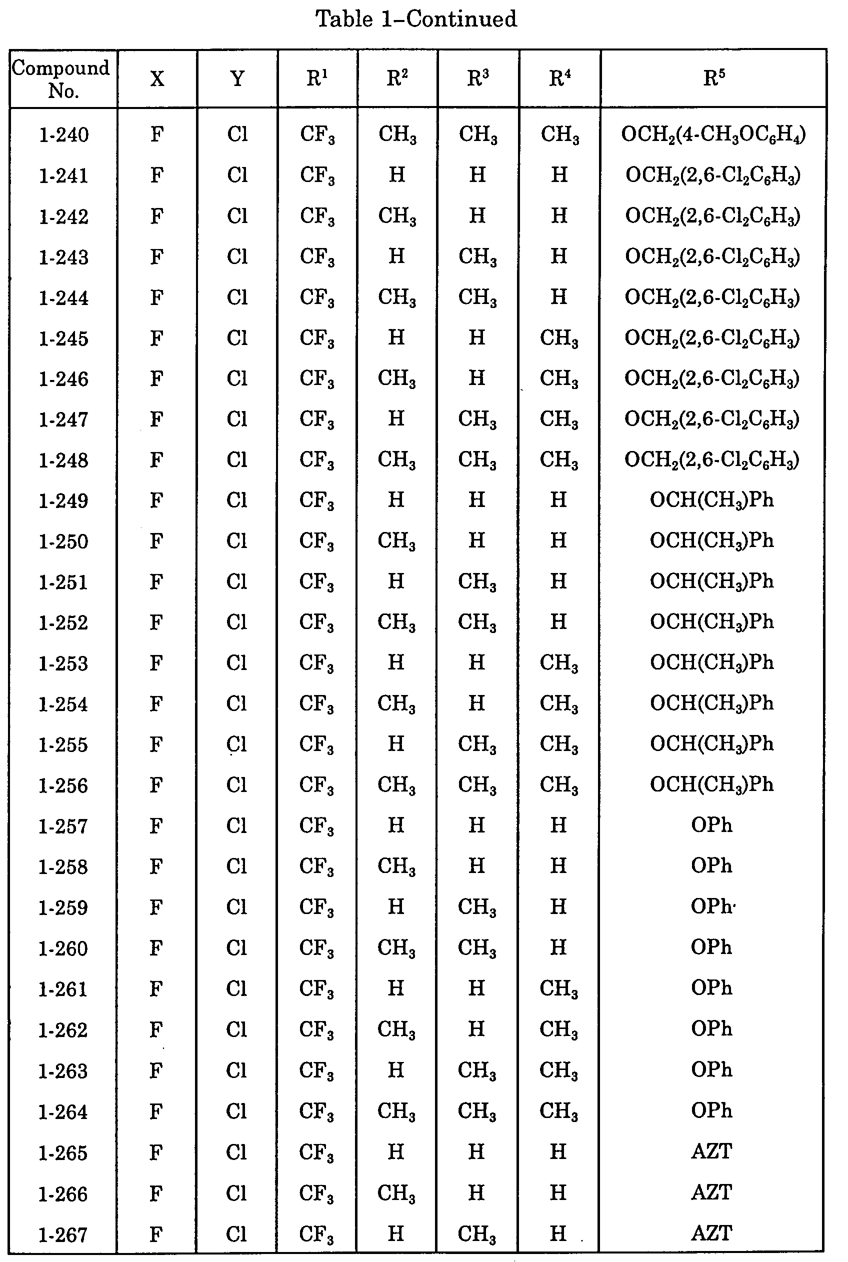

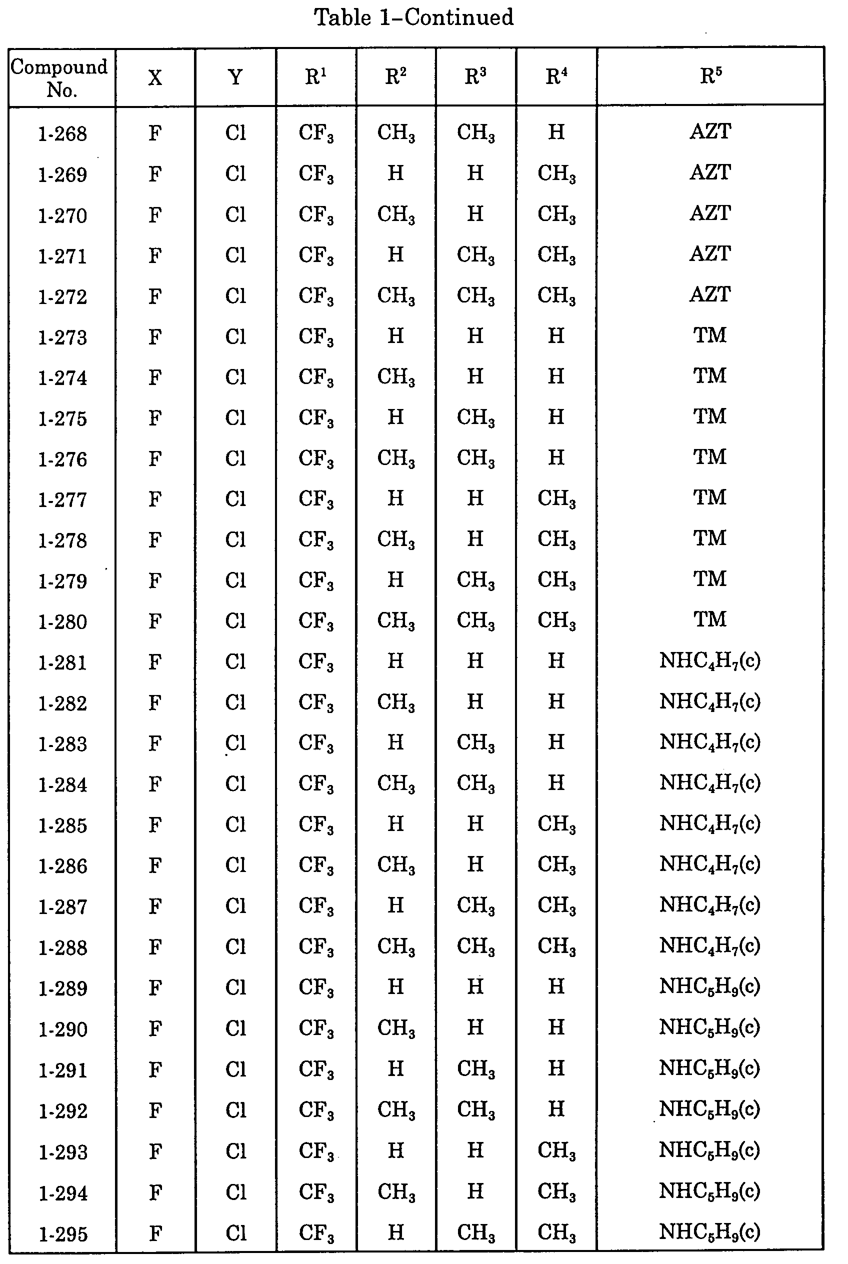

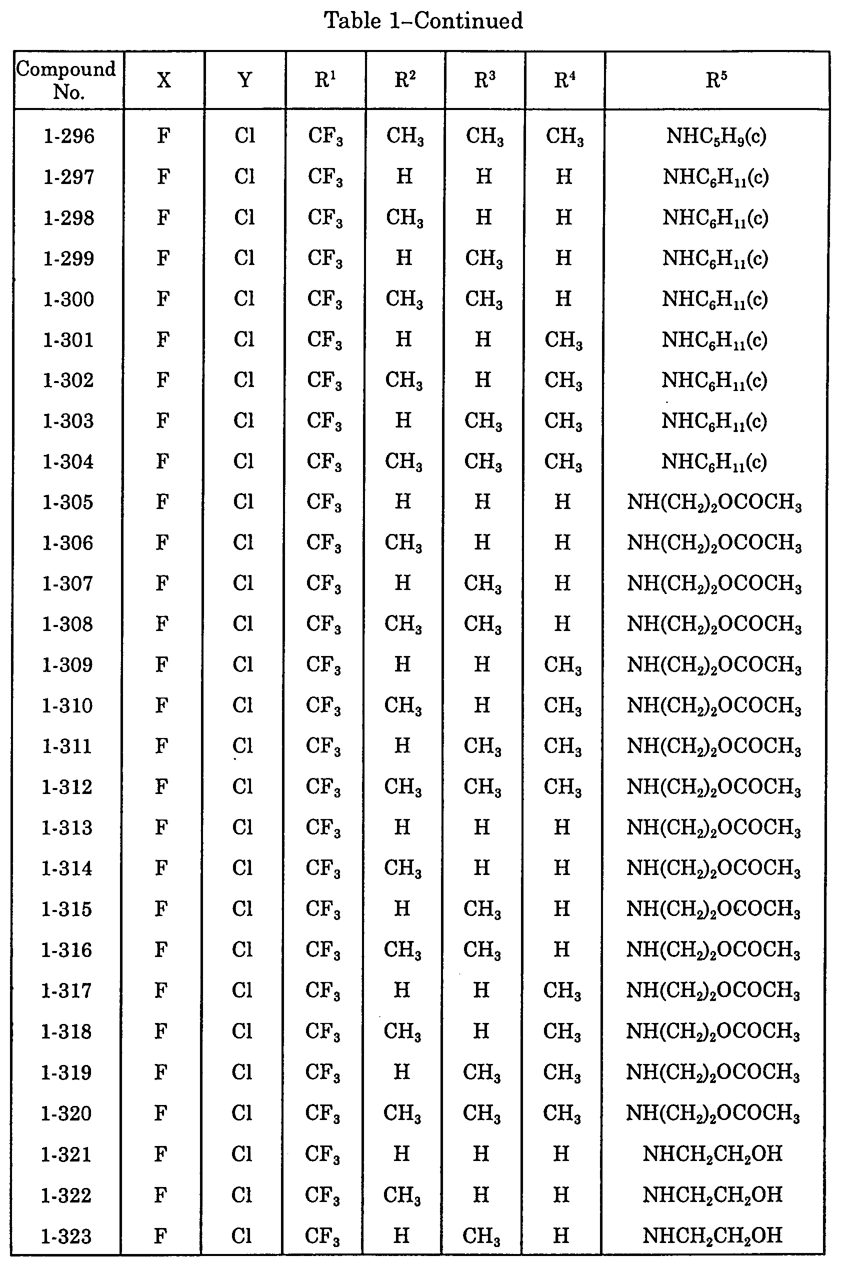

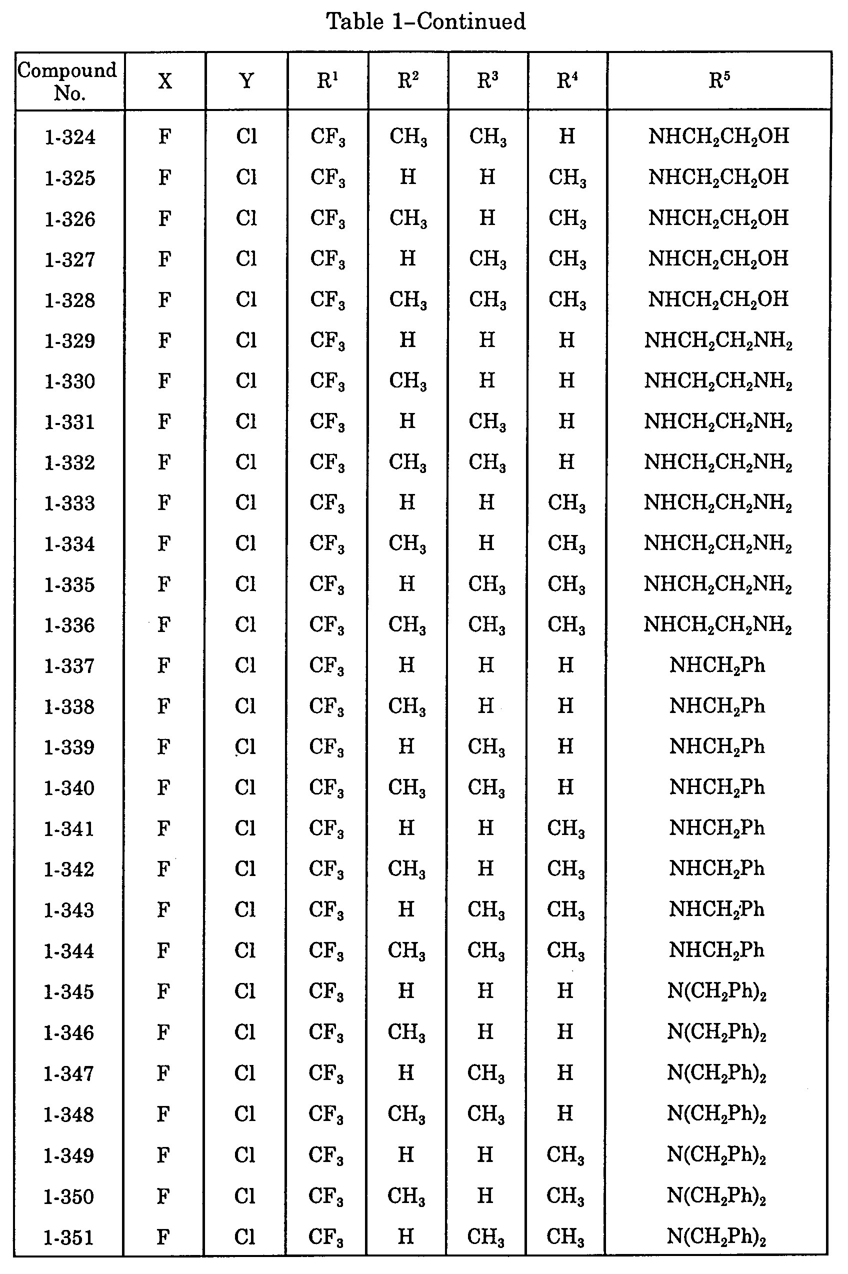

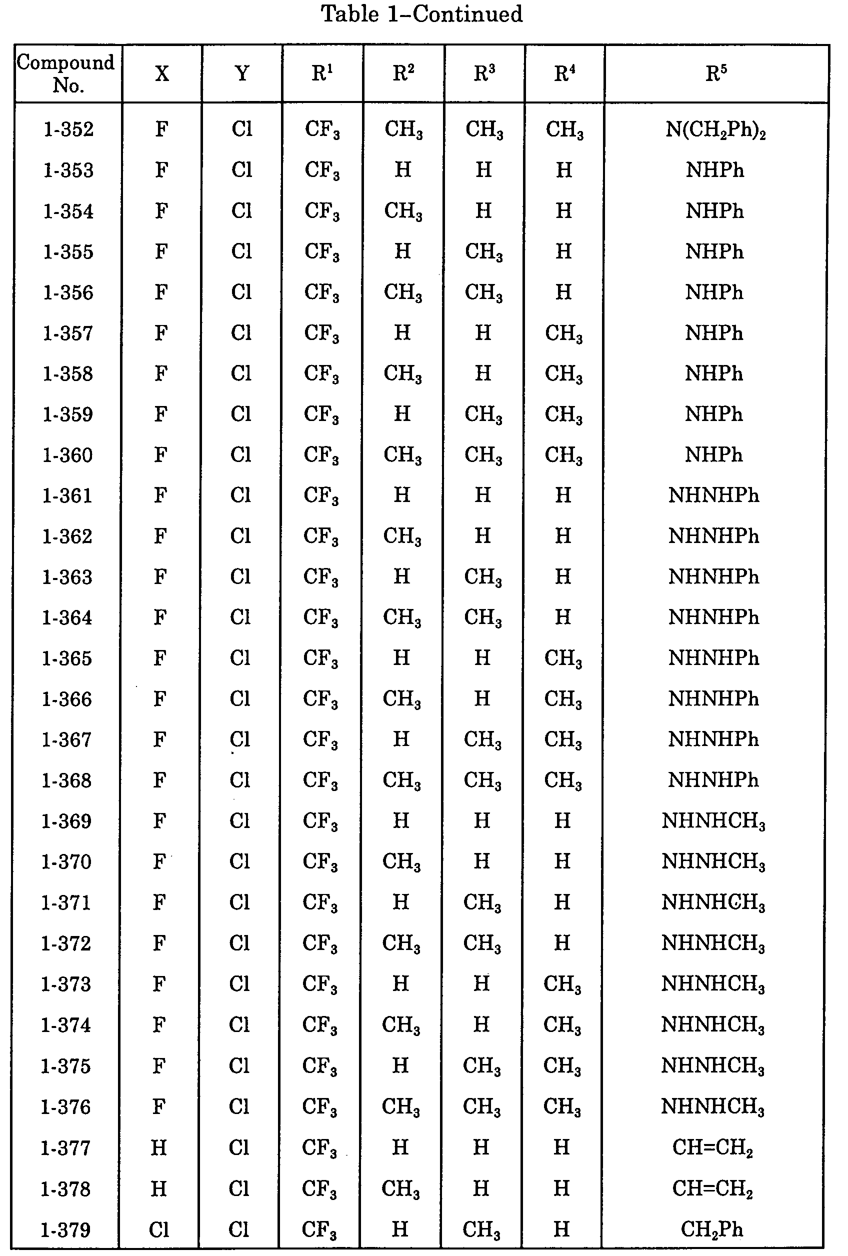

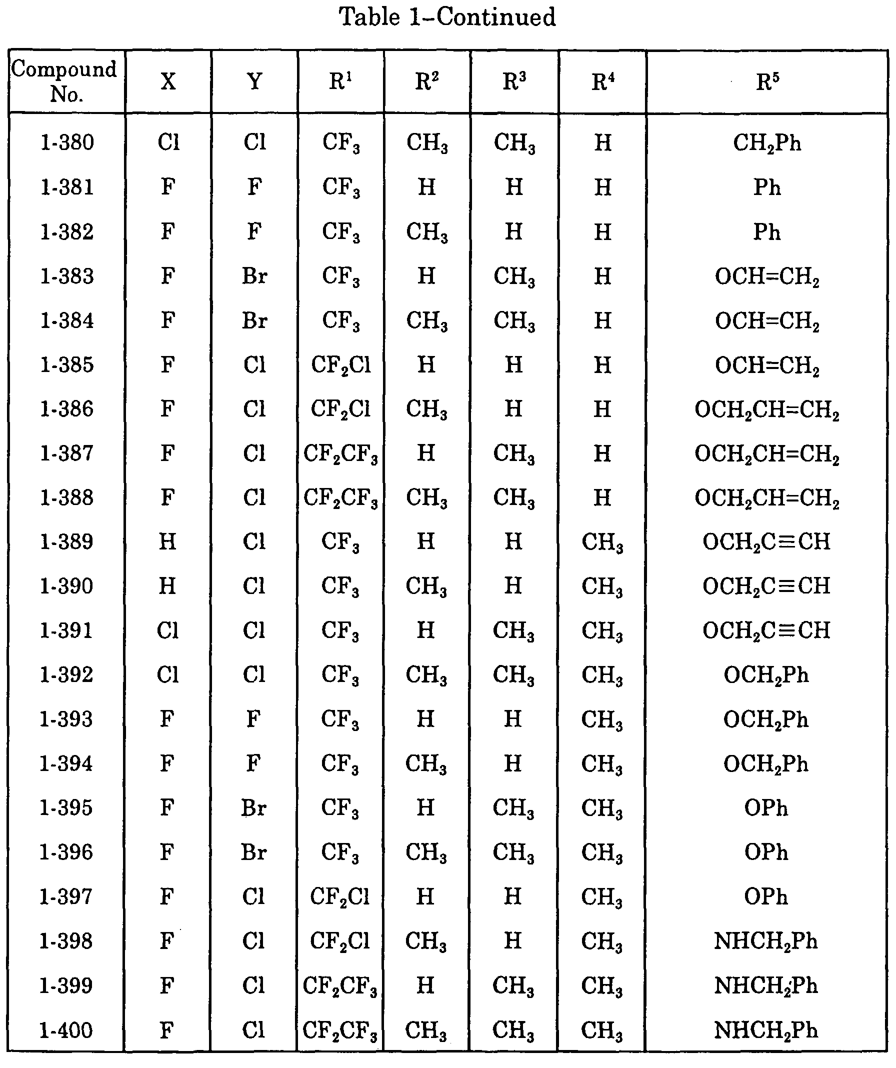

In Table 1, "Ph", "AZT", "TM", "C₄H₇(c)", "C₅H₉(c)", and "C₆H₁₁(c)" represent phenyl, azetidin-1-yl, thiomorpholin-1-yl, cyclobutyl, cyclopentyl, and cyclohexyl, respectively.

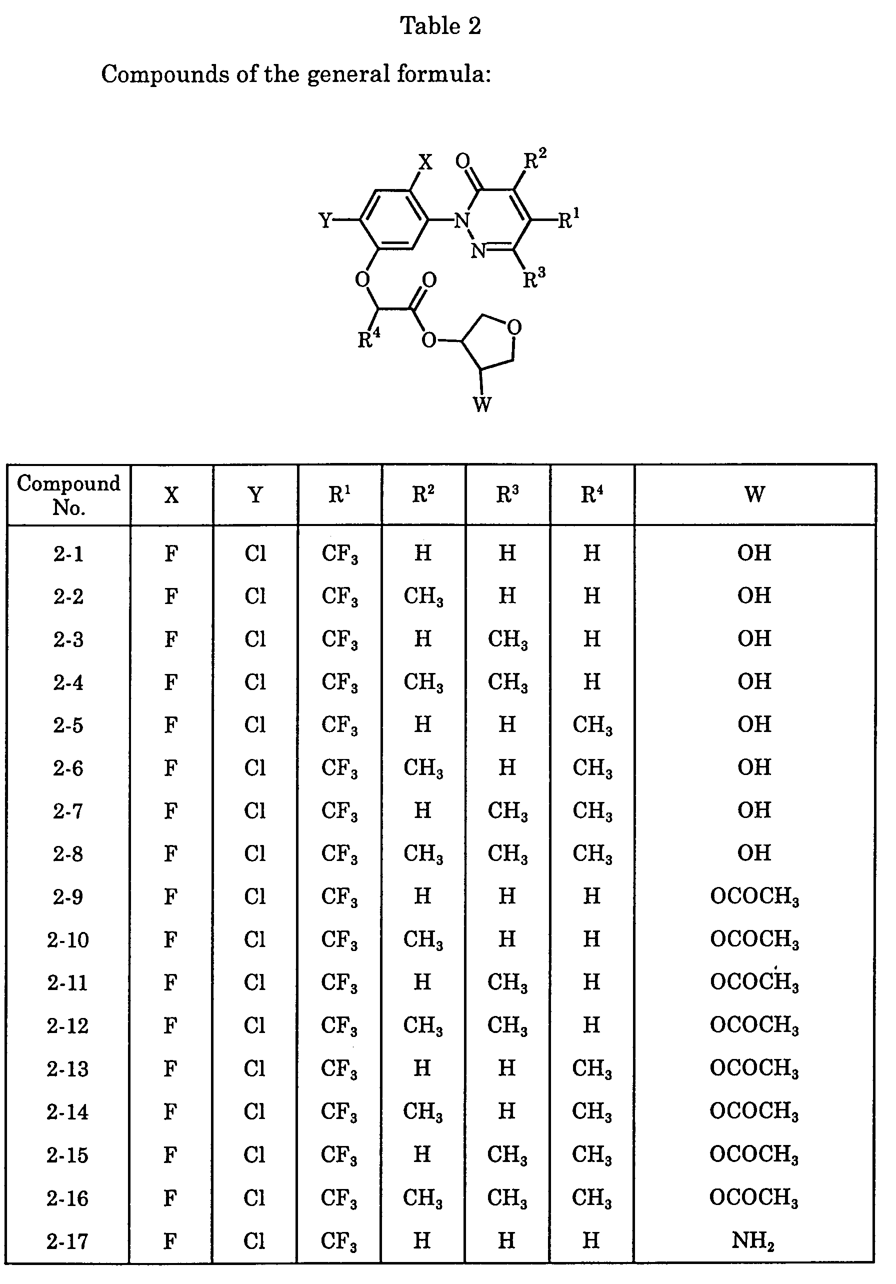

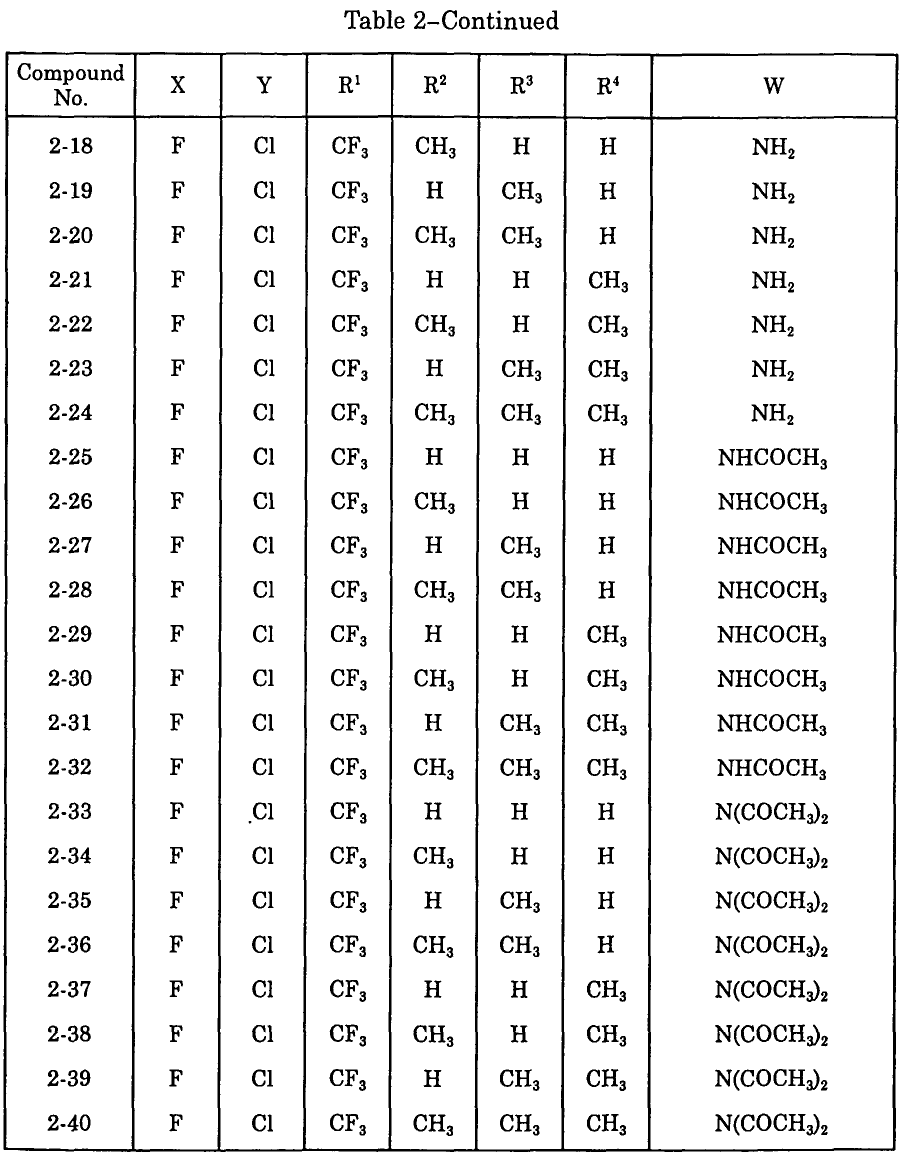

The following will describe a reference production example for the above-described compound [X].

### Reference Production Example

First, 32.3 g of 5-amino-2-chloro-4-fluorophenol (this compound was produced by the process disclosed in the published specification of European Patent Application, EP-61741-A) was mixed with 150 ml of concentrated hydrochloric acid, and the mixture was stirred at 50°C for 30 minutes. To this mixture was added dropwise at 0°C over 10 minutes a solution of 15 g of sodium nitrite dissolved in 40 ml of water. The mixture was stirred at 0°C for 1 hour and then cooled to -50°C. To this mixture was rapidly added dropwise at -50°C a solution of 132 g of tin (II) chloride dissolved in 132 g of concentrated hydrochloric acid, and the mixture was slowly returned to room temperature and then further stirred at room temperature for 1 hour. The resulting solids were collected by filtration, and dried at 80°C under reduced pressure, which afforded 75 g of crude crystals of 2-fluoro-4-chloro-5-hydroxyphenylhydrazine hydrochloride.
¹H-NMR (DMSO-d₆, TMS, δ (ppm)): 3-5 (2H, br), 6.73 (1H, d), 7.22 (1H, d), 8.20 (1H, s), 9-11 (2H, br).

Then, 49.2 g of sodium acetate and 40.5 g of 1,1-dibromo-3,3,3-trifluoroacetone were dissolved in 400 ml of water, and the solution was heated at 80° to 90°C for 40 minutes. This solution was cooled to 0°C, to which 75 g of the crude crystals of 2-fluoro-4-chloro-5-hydroxyphenylhydrazine hydrochloride was added. The mixture was stirred at room temperature for 70 minutes, and the resulting crystals were collected by filtration, and dried under reduced pressure, which afforded 35.4 g of 3,3,3-trifluoro-2-oxopropanal 1-(4-chloro-2-fluoro-5-hydroxyphenylhydrazone).
¹H-NMR (300 MHz, CDCl₃, TMS, δ (ppm)): 5.49 (1H, s), 7.15 (1H, d, J = 10.5 Hz), 7.24 (1H, d, J = 7.4 Hz), 7.38 (1H, q, J = 1.8 Hz), 8.75 (1H, s).

Then, 12.9 g of 3,3,3-trifluoro-2-oxopropanal 1-(4-chloro-2-fluoro-5-hyroxyphenylhydrazone) and 22.3 g of (carbethoxyethylidene)triphenylphosphorane were dissolved in 110 ml of THF, and the solution was heated under reflux for 3 hours. The solvent was evaporated under reduced pressure, and the residue was subjected to silica gel column chromatography, which afforded 8.8 g of 2-(2-fluoro-4-chloro-5-hydroxyphenyl)-4-methyl-5-trifluoromethylpyridazin-3-one.

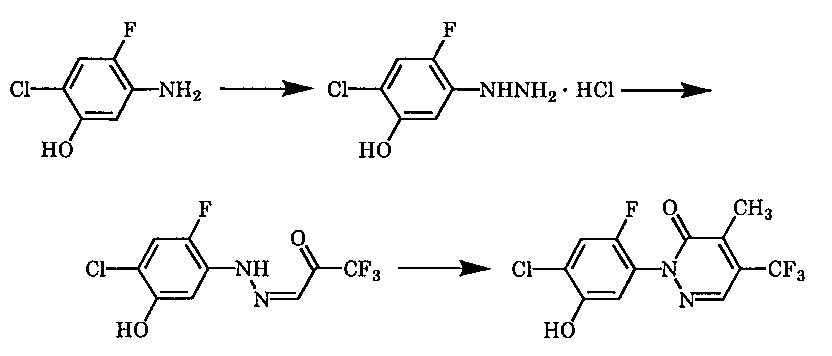

The following will describe formulation examples, in which the present compounds are designated by their compound numbers shown in Tables 1 and 2, and parts are by weight.

### Formulation Example 1

Fifty parts of each of the present compounds 1-1 to 1-400 and 2-1 to 2-40, 3 parts of calcium lignin sulfonate, 2 parts of sodium laurylsulfate, and 45 parts of synthetic hydrated silicon oxide are well pulverized and mixed to give a wettable powder for each compound.

### Formulation Example 2

Ten parts of each of the present compounds 1-1 to 1-400 and 2-1 to 2-40, 14 parts of polyoxyethylene styryl phenyl ether, 6 parts of calcium dodecylbenzenesulfonate, 35 parts of xylene, and 35 parts of cyclohexanone are well mixed to give an emulsifiable concentrate for each compound.

### Formulation Example 3

Two parts of the present compounds 1-1 to 1-400 and 2-1 to 2-40, 2 parts of synthetic hydrated silicon oxide, 2 parts of calcium lignin sulfonate, 30 parts of bentonite, and 64 parts of kaolin clay are well pulverized and mixed. The mixture is well kneaded with the addition of water, and then granulated and dried to give a granule for each compound.

### Formulation Example 4

Twenty-five parts of the present compounds 1-1 to 1-400 and 2-1 to 2-40, 50 parts of 10% aqueous solution of polyvinyl alcohol, and 25 parts of water are mixed and then wet pulverized until the mean particle size comes to 5 µm or smaller to give a flowable for each compound.

The following test examples will demonstrate that the present compounds are useful as the active ingredients of herbicides. The present compounds are designated by their compound numbers shown in Tables 1 and 2.

The herbicidal activity and phytotoxicity are evaluated at 6 levels with indices of 0 to 5, i.e., shown by numeral "0", "1", "2", "3", "4", or "5", wherein "0" means that there was no or little difference in the degree of germination or growth between the treated plants (i.e., weeds and crops) and the untreated plants at the time of examination, and "5" means that the test plants died completely or their germination or growth was completely inhibited. The herbicidal activity is excellent when ranked at "4" or "5", but insufficient when ranked at "3" or lower. The phytotoxicity is not significant for practical use when ranked at "0" or "1", but not allowed when ranked at "2" or higher.

### Test Example 1 Foliar treatment on upland fields

Cylindrical plastic pots each having a diameter of 10 cm and a depth of 10 cm were filled with soil, and then seeded with barnyardgrass (*Echinochloa crus-galli*), ivyleaf morningglory (*Ipomoea hederacea*), and velvetleaf (*Abutilon theophrasti*). These test plants were grown in a greenhouse for 19 days. Each of the test compounds listed below was formulated into an emulsifiable concentrate according to Formulation Example 2, and then diluted in its prescribed amount with water containing a spreading agent. The dilution was uniformly sprayed over the foliage of the test plants with a sprayer at a rate of 1000 liters per hectare After the application, the test plants were grown in the greenhouse for 19 days, and the herbicidal activity was examined. The results are shown in Table 3.

**Table 3**

| Test compound | Application amount of active ingredient (g/ha) | Herbicidal activity | | |
|---|---|---|---|---|
| | | barnyardgrass | ivyleaf morningglory | velvetleaf |
| 1-54 | 500 | 5 | 5 | 5 |
| 1-58 | 500 | 5 | 5 | 5 |
| 1-66 | 500 | 5 | 5 | 5 |
| 1-70 | 500 | 5 | 5 | 5 |
| 1-98 | 500 | 5 | 5 | 5 |
| 1-102 | 500 | 5 | 5 | 5 |
| 1-194 | 500 | 5 | 5 | 5 |
| 1-198 | 500 | 5 | 5 | 5 |
| 1-258 | 500 | 5 | 5 | 5 |
| 1-262 | 500 | 5 | 5 | 5 |
| 1-266 | 500 | 5 | 5 | 5 |
| 1-274 | 500 | 5 | 5 | 5 |
| 1-306 | 500 | 5 | 5 | 5 |
| 1-322 | 500 | 5 | 5 | 5 |
| 2-2 | 500 | 5 | 5 | 5 |
| 2-10 | 500 | 5 | 5 | 5 |

### Test Example 2 Foliar treatment on upland fields

Cylindrical plastic pots each having a diameter of 10 cm and a depth of 10 cm were filled with soil, and then seeded with barnyardgrass (*Echinochloa crus-galli*), ivyleaf morningglory (*Ipomoea hederacea*), and velvetleaf (*Abutilon theophrasti*). These test plants were grown in a greenhouse for 19 days. Each of the test compounds listed below was formulated into an emulsifiable concentrate according to Formulation Example 2, and then diluted in its prescribed amount with water containing a spreading agent. The dilution was uniformly sprayed over the foliage of the test plants with a sprayer at a rate of 1000 liters per hectare After the application, the test plants were grown in the greenhouse for 19 days, and the herbicidal activity was examined. The results are shown in Table 4.

**Table 4**

| Test compound | Application amount of active ingredient (g/ha) | Herbicidal activity | | |
|---|---|---|---|---|
| | | barnyardgrass | ivyleaf morningglory | velvetleaf |
| 1-58 | 32 | 5 | 5 | 5 |
| 1-66 | 32 | 5 | 5 | 5 |
| 1-70 | 32 | 5 | 5 | 5 |
| 1-102 | 32 | 5 | 5 | 5 |
| 1-194 | 32 | 4 | 5 | 5 |
| 1-198 | 32 | 5 | 5 | 5 |
| 1-258 | 32 | 5 | 5 | 5 |
| 1-262 | 32 | 5 | 5 | 5 |
| 2-2 | 32 | 4 | 5 | 5 |
| 2-10 | 32 | 5 | 5 | 5 |

### Test Example 3 Soil surface treatment on upland fields

Cylindrical plastic pots each having a diameter of 10 cm and a depth of 10 cm were filled with soil, and then seeded with barnyardgrass (*Echinochloa crus-galli*), ivyleaf morningglory (*Ipomoea hederacea*), and velvetleaf (*Abutilon theophrasti*). Each of the test compounds listed below was formulated into an emulsifiable concentrate according to Formulation Example 2, and then diluted in its prescribed amount with water. The dilution was uniformly sprayed over the soil surface in the pots with a sprayer at a rate of 1000 liters per hectare After the application, the test plants were grown in the greenhouse for 19 days, and the herbicidal activity was examined. The results are shown in Table 5.

**Table 5**

| Test compound | Application amount of active ingredient (g/ha) | Herbicidal activity | | |
|---|---|---|---|---|
| | | barnyardgrass | ivyleaf morningglory | velvetleaf |
| 1-58 | 500 | 5 | 5 | 5 |
| 1-66 | 500 | 5 | 5 | 5 |
| 1-70 | 500 | 5 | 5 | 5 |
| 1-102 | 500 | 5 | 5 | 5 |
| 1-194 | 500 | 5 | 5 | 5 |
| 1-198 | 500 | 5 | 5 | 5 |
| 1-258 | 500 | 5 | 5 | 5 |
| 1-262 | 500 | 5 | 5 | 5 |
| 1-266 | 500 | 5 | 5 | 5 |
| 1-322 | 500 | 5 | 5 | 5 |
| 2-2 | 500 | 5 | 5 | 5 |
| 2-10 | 500 | 5 | 5 | 5 |

### Test Example 4 Flooding treatment on paddy fields

Cylindrical plastic pots each having a diameter of 9 cm and a depth of 11 cm were filled with soil, and then seeded with barnyardgrass (*Echinochloa oryzicola*). These pots were flooded to form paddy fields, and the test plants were grown in a greenhouse for 7 days. Each of the test compounds listed below was formulated into an emulsifiable concentrate according to Formulation Example 2, and then diluted in its prescribed amount with water. The dilution was applied to the water surface in the pots at a rate of 50 liters per are. After the application, the test plants were grown in the greenhouse for 16 days, and the herbicidal activity was examined. The results are shown in Table 6.

**Table 6**

| Test compound | Application amount of active ingredient (g/ha) | Herbicidal activity |
|---|---|---|
| | | barnyardgrass |
| 1-54 | 250 | 5 |
| 1-58 | 250 | 5 |
| 1-66 | 250 | 5 |
| 1-70 | 250 | 5 |
| 1-98 | 250 | 5 |
| 1-102 | 250 | 5 |
| 1-194 | 250 | 5 |
| 1-198 | 250 | 5 |
| 1-258 | 250 | 5 |
| 1-262 | 250 | 5 |
| 1-266 | 250 | 4 |
| 1-274 | 250 | 5 |
| 1-306 | 250 | 5 |
| 1-322 | 250 | 5 |
| 2-2 | 250 | 5 |
| 2-10 | 250 | 5 |

### Test Example 5 Foliar treatment on upland fields

Plastic pots each having an area of 25 x 18 cm² and a depth of 7 cm were filled with soil, and then seeded with corn (*Zea mays*), soybean (*Glycine max*), common cocklebur (*Xanthium pensylvanicum*), ivyleaf morningglory (*Ipomoea hederacea*), common ragweed (*Ambrosia artemisiifolia*), and common lambsquarters (*Chenopodium album*). These test plants were grown for 21 days. The test compound described below was formulated into an emulsifiable concentrate according to Formulation Example 2, and then diluted in its prescribed amount with water. The dilution was uniformly sprayed over the foliage of the test plants with a sprayer at a rate of 1000 liters per hectare At this time, the weeds and crops, although their growth was at different levels depending upon the grass species, were in the one- to four-leave stage and had different grass heights of 5 to 20 cm. After 24 days from the application, the herbicidal activity and phytotoxicity were examined. The results are shown in Table 7. The test was carried out in a greenhouse over the whole period.

**Table 7**

| Test compound | Application amount of active ingredient (g/ha) | Phytotoxicity on crops | | Herbicidal activity | | | |
|---|---|---|---|---|---|---|---|
| | | corn | soybean | common cocklebur | ivyleaf morningglory | common ragweed | common lambsquarters |
| 1-194 | 63 | 1 | 1 | 5 | 5 | 5 | 4 |

### Test Example 6 Foliar treatment on upland fields

Plastic pots each having an area of 16 x 11 cm² and a depth of 7 cm were filled with soil, and then seeded with wheat (*Triticum aestivum*), pale smartweed (*Polygonum lapathifolium*), and catchweed (*Galium ararine*). These test plants were grown for 23 days. The test compound described below was formulated into an emulsifiable concentrate according to Formulation Example 2, and then diluted in its prescribed amount with water. The dilution was uniformly sprayed over the foliage of the test plants with a sprayer at a rate of 1000 liters per hectare At this time, the weeds and crops, although their growth was at different levels depending upon the grass species, were in the one- to four-leave stage and had different grass heights of 5 to 15 cm. After 24 days from the application, the herbicidal activity and phytotoxicity were examined. The results are shown in Table 8. The test was carried out in a greenhouse over the whole period.

**Table 8**

| Test compound | Application amount of active ingredient (g/ha) | Phytotoxicity on crops | Herbicidal activity | |
|---|---|---|---|---|
| | | wheat | pale smartweed | catchweed |
| 1-258 | 63 | 0 | 4 | 5 |

### Test Example 7 Foliar treatment on upland fields

Plastic pots each having an area of 25 x 18 cm² and a depth of 7 cm were filled with soil, and then seeded with rice (*Oryza sativa*) and barnyardgrass (*Echinochloa crus-galli*). These test plants were grown for 15 days. The test compound described below was formulated into an emulsifiable concentrate according to Formulation Example 2, and then diluted in its prescribed amount with water. The dilution was uniformly sprayed over the foliage of the test plants with a sprayer at a rate of 1000 liters per hectare At this time, the weeds and crops, although their growth was at different levels depending upon the grass species, were in the one- to three-leave stage and had different grass heights of 8 to 15 cm. After 24 days from the application, the herbicidal activity and phytotoxicity were examined. The results are shown in Table 9. The test was carried out in a greenhouse over the whole period.

**Table 9**

| Test compound | Application amount of active ingredient (g/ha) | Phytotoxicity on crops | Herbicidal activity |
|---|---|---|---|
| | | rice | barnyardgrass |
| 2-10 | 63 | 0 | 5 |

## Claims

1. A pyridazin-3-one derivative of general formula (1): 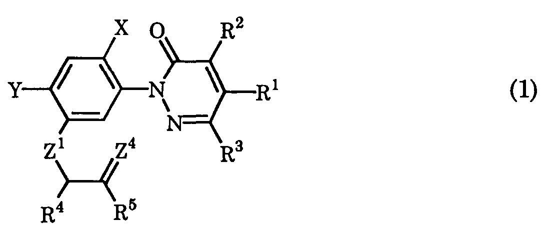 wherein
R¹ is C₁-C₃ haloalkyl;
R² and R³ are the same or different, and are independently hydrogen, C₁-C₃ alkyl, C₁-C₃ haloalkyl, or C₁-C₃ alkoxy C₁-C₃ alkyl;
X is hydrogen or halogen;
Y is halogen, nitro, cyano, or trihalomethyl;
Z¹ is oxygen, sulfur, CH₂, or NH;
Z⁴ is oxygen or sulfur;
R⁴ is hydrogen, C₁-C₆ alkyl, or C₃-C₆ cycloalkyl; and
R⁵ is C₂-C₆ alkenyl, C₂-C₆ haloalkenyl, C₃-C₆ alkynyl, C₃-C₆ haloalkynyl, optionally substituted benzyl, optionally substituted phenyl, C₂-C₆ alkenyloxy, C₂-C₆ haloalkenyloxy, C₃-C₆ alkynyloxy, C₃-C₆ haloalkynyloxy, C₁-C₆ alkylthio, C₁-C₆ haloalkylthio, C₂-C₆ alkenylthio, C₂-C₆ haloalkenylthio, (C₁-C₆ alkyl)thio C₁-C₆ alkyl, C₃-C₆ alkynylthio, C₃-C₆ haloalkynylthio, C₃-C₈ cycloalkylthio, C₃-C₈ cyclohaloalkylthio, (C₃-C₈ cycloalkyl) C₁-C₆ akylthio, di(C₁-C₆ alkyl)C=NO, optionally substituted benzylthio, optionally substituted phenylthio, hydroxy C₂-C₆ alkoxy, amino C₂-C₆ alkoxy, (C₁-C₆ alkyl)carbonyloxy C₂-C₆ alkoxy, (C₁-C₆ alkyl)carbonylamino C₂-C₆ alkoxy, optionally substituted benzyloxy, optionally substituted phenoxy, optionally substituted furyloxy, optionally substituted furyl C₁-C₆ alkyloxy, optionally substituted thienyloxy, optionally substituted thienyl C₁-C₆ alkyloxy, optionally substituted pyrrolyloxy, optionally substituted pyrrolyloxy C₁-C₆ alkyloxy, optionally substituted imidazolyloxy, optionally substituted imidazolyl C₁-C₆ alkyloxy, optionally substituted pyrazolyloxy, optionally substituted pyrazolyl C₁-C₆ alkyloxy, optionally substituted thiazolyloxy, optionally substituted thiazolyl C₁-C₆ alkyloxy, optionally substituted oxazolyloxy, optionally substituted oxazolyl C₁-C₆ alkyloxy, optionally substituted isothiazolyloxy, optionally substituted isothiazolyl C₁-C₆ alkyloxy, optionally substituted isoxazolyloxy, optionally substituted isoxazolyl C₁-C₆ alkyloxy, optionally substituted pyridyloxy, optionally substituted pyridyl C₁-C₆ alkyloxy, optionally substituted pyrazinyloxy, optionally substituted pyrazinyl C₁-C₆ alkyloxy, optionally substituted pyrimidinyloxy, optionally substituted pyrimidinyl C₁-C₆ alkyloxy, optionally substituted pyridazinyloxy, optionally substituted pyridazinyl C₁-C₆ alkyloxy, optionally substituted indolidinyloxy, optionally substituted indolidinyl C₁-C₆ alkyloxy, optionally substituted indolyloxy, optionally substituted indolyl C₁-C₆ alkyloxy, optionally substituted indazolyloxy, optionally substituted indazolyl C₁-C₆ alkyloxy, optionally substituted quinolyloxy, optionally substituted quinolyl C₁-C₆ alkyloxy, optionally substituted isoquinolyloxy, or optionally substituted isoquinolyl C₁-C₆ alkyloxy, or
a group of -NR⁶R⁷ wherein R⁶ is hydrogen, C₃-C₈ cycloalkyl, (C₁-C₆ alkyl) carbonyloxy C₂-C₆ alkyl, (C₁-C₆ alkyl)carbonylamino C₂-C₆ alkyl, hydroxy C₂-C₆ alkyl, amino C₂-C₆ alkyl, optionally substituted benzyl, or optionally substituted phenyl, and R⁷ is C₃-C₈ cycloalkyl, (C₁-C₆ alkyl)carbonyloxy C₂-C₆ alkyl, (C₁-C₆ alkyl)carbonylamino C₂-C₆ alkyl, hydroxy C₂-C₆ alkyl, amino C₂-C₆ alkyl, optionally substituted benzyl, or optionally substituted phenyl; or R⁶ and R⁷ are taken together to form trimethylene or ethylenethioethylene, or
a group of -NHNHR⁸ wherein R⁸ is hydrogen, C₁-C₆ alkyl, optionally substituted phenyl, or optionally substituted benzyl, or
a group of general formula (2): 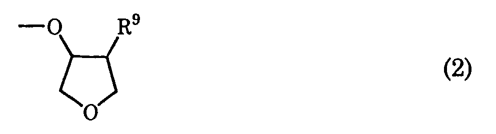 wherein R⁹ is hydrogen, hydroxyl, or a group of -O-COR¹⁰, and R¹⁰ is C₁-C₆ alkyl, C₁-C₆ haloalkyl, C₂-C₆ alkenyl, C₃-C₈ cycloalkyl, optionally substituted phenyl, optionally substituted benzyl, C₁-C₆ alkoxy, or C₁-C₆ alkylamino.

2. A pyridazin-3-one derivative according to claim 1, wherein Z¹ is oxygen, sulfur, or NH, and R⁵ is C₂-C₆ alkenyl, C₂-C₆ haloalkenyl, C₃-C₆ alkynyl, C₃-C₆ haloalkynyl, optionally substituted benzyl, optionally substituted phenyl, C₁-C₆ alkylthio, C₁-C₆ haloalkylthio, C₂-C₆ alkenylthio, C₂-C₆ haloalkenylthio, (C₁-C₆ alkyl)thio C₁-C₆ alkyl, C₃-C₆ alkynylthio, C₃-C₆ haloalkynylthio, C₃-C₈ cycloalkylthio, C₃-C₈ cyclohaloalkylthio, (C₃-C₈ cycloalkyl) C₁-C₆ alkylthio, di(C₁-C₆ alkyl)C=NO, optionally substituted benzylthio, optionally substituted phenylthio, hydroxy C₂-C₆ alkoxy, amino C₂-C₆ alkoxy, (C₁-C₆ alkyl)carbonyloxy C₂-C₆ alkoxy, (C₁-C₆ alkyl)carbonylamino C₂-C₆ alkoxy, optionally substituted furyloxy, optionally substituted furyl C₁-C₆ alkyloxy, optionally substituted thienyloxy, optionally substituted thienyl C₁-C₆ alkyloxy, optionally substituted pyrrolyloxy, optionally substituted pyrrolyloxy C₁-C₆ alkyloxy, optionally substituted imidazolyloxy, optionally substituted imidazolyl C₁-C₆ alkyloxy, optionally substituted pyrazolyloxy, optionally substituted pyrazolyl C₁-C₆ alkyloxy, optionally substituted thiazolyloxy, optionally substituted thiazolyl C₁-C₆ alkyloxy, optionally substituted oxazolyloxy, optionally substituted oxazolyl C₁-C₆ alkyloxy, optionally substituted isothiazolyloxy, optionally substituted isothiazolyl C₁-C₆ alkyloxy, optionally substituted isoxazolyloxy, optionally substituted isoxazolyl C₁-C₆ alkyloxy, optionally substituted pyridyloxy, optionally substituted pyridyl C₁-C₆ alkyloxy, optionally substituted pyrazinyloxy, optionally substituted pyrazinyl C₁-C₆ alkyloxy, optionally substituted pyrimidinyloxy, optionally substituted pyrimidinyl C₁-C₆ alkyloxy, optionally substituted pyridazinyloxy, optionally substituted pyridazinyl C₁-C₆ alkyloxy, optionally substituted indolidinyloxy, optionally substituted indolidinyl C₁-C₆ alkyloxy, optionally substituted indolyloxy, optionally substituted indolyl C₁-C₆ alkyloxy, optionally substituted indazolyloxy, optionally substituted indazolyl C₁-C₆ alkyloxy, optionally substituted quinolyloxy, optionally substituted quinolyl C₁-C₆ alkyloxy, optionally substituted isoquinolyloxy, optionally substituted isoquinolyl C₁-C₆ alkyloxy, a group of -NR⁶R⁷ wherein R⁶ and R⁷ are as defined in claim 1, a group of -NHNHR⁸ wherein R⁸ is as defined in claim 1, or a group of general formula (2) as depicted in claim 1.

3. A pyridazin-3-one derivative according to claim 1 or 2, wherein a substituent referred to by "optionally substituted" is C₁-C₄ alkyl, C₁-C₄ haloalkyl, C₁-C₄ alkoxy, (C₁-C₄ alkyl)carbonyloxy, (C₁-C₄ alkoxy)carbonyl, C₁-C₄ haloalkoxy, (C₁-C₄ haloalkyl)carbonyloxy, (C₁-C₄ haloalkoxy)carbonyl, halogen, nitro, cyano, hydroxyl, amino, or acetyl.

4. A pyridazin-3-one derivative according to claim 1, wherein R¹ is trifluoromethyl, R² is methyl or hydrogen, R³ is hydrogen, X is fluorine, Y is chlorine, R⁴ is hydrogen or methyl, and R⁵ is C₂-C₆ alkenyloxy C₂-C₆ haloalkenyloxy, C₃-C₆ alkynyloxy, C₃-C₆ haloalkynyloxy, optionally substituted benzyloxy, or optionally substituted phenoxy.

5. A pyridazin-3-one derivative according to claim 4, wherein a substituent referred to by "optionally substituted" is C₁-C₄ alkyl, C₁-C₄ haloalkyl, C₁-C₄ alkoxy, (C₁-C₄ alkyl)carbonyloxy, (C₁-C₄ alkoxy)carbonyl, C₁-C₄ haloalkoxy, (C₁-C₄ haloalkyl)carbonyloxy, (C₁-C₄ haloalkoxy)carbonyl, halogen, nitro, cyano, hydroxyl, amino, or acetyl.

6. A pyridazin-3-one derivative according to any one of claims 1 to 5, wherein Z¹ and Z⁴ are both oxygen.

7. A pyridazin-3-one derivative according to claim 4 or 6, wherein R⁵ is C₂-C₆ alkenyloxy, C₃-C₆ alkynyloxy, benzyloxy, or phenoxy.

8. A pyridazin-3-one derivative according to claim 4 or 6, wherein R⁵ is C₂-C₆ alkenyloxy.

9. A pyridazin-3-one derivative according to claim 4 or 6, wherein R⁵ is phenoxy.

10. A pyridazin-3-one derivative according to claim 4 or 6, wherein R⁵ is benzyloxy.

11. A pyridazin-3-one derivative according to any one of claims 4 to 10, wherein R² is methyl.

12. A pyridazin-3-one derivative according to claim 2, 3, or 6, wherein Y is halogen, R¹ is trifluoromethyl, R² is hydrogen or methyl, R³ is hydrogen, and R⁴ is hydrogen or methyl.

13. A pyridazin-3-one derivative according to claim 2, 3, or 6, wherein X is fluorine, Y is halogen, R¹ is trifluoromethyl, R² is hydrogen or methyl, R³ is hydrogen, and R⁴ is hydrogen or methyl.

14. A pyridazin-3-one derivative according to claim 2, 3, or 6, wherein X is fluorine, Y is chlorine, R¹ is trifluoromethyl, R² is hydrogen or methyl, R³ is hydrogen, and R⁴ is hydrogen or methyl.

15. A pyridazin-3-one derivative according to claim 2, 3, or 6, wherein X is fluorine, Y is chlorine, R¹ is trifluoromethyl, R² is hydrogen or methyl, R³ is hydrogen, and R⁴ is methyl.

16. A pyridazin-3-one derivative according to claim 2, 12, 13, 14, or 15, wherein R⁵ is NHR⁷, and R⁷ is (C₁-C₆ alkyl)carbonyloxy C₂-C₆ alkyl, hydroxy C₂-C₆ alkyl, benzyl, or phenyl.

17. A pyridazin-3-one derivative according to claim 2, 12, 13, 14, or 15, wherein R⁵ is azetidin-1-yl or thiomorpholin-1-yl.

18. A pyridazin-3-one derivative according to claim 2, 12, 13, 14, or 15, wherein R⁵ is a group of general formula (2) as depicted in claim 1, and R⁹ is hydroxyl or (C₁-C₆ alkyl)carbonyloxy.

19. A pyridazin-3-one derivative according to claim 1 or 2, wherein R¹ is CF₂Z², and Z² is hydrogen, fluorine, chlorine, or trifluoromethyl.

20. A pyridazin-3-one derivative according to claim 1 or 2, wherein R¹ is trifluoromethyl.

21. A composition comprising a pyridazin-3-one derivative as set forth in any one of claims 1 to 20 as an active ingredient.

22. A method for controlling weeds, which comprises applying an effective amount of a pyridazin-3-one derivative as set forth in any one of claims 1 to 20, to the weeds or to a place where the weeds are growing or will grow.

23. Use of a pyridazin-3-one derivative as set forth in any one of claims 1 to 20 as a herbicide.
